(19) Europäisches Patentamt
European Patent Office
Office européen des brevets

(11) Veröffentlichungsnummer: **0 414 067 A2**

# (12) EUROPÄISCHE PATENTANMELDUNG

(21) Anmeldenummer: **90115373.4**

(22) Anmeldetag: **10.08.90**

(51) Int. Cl.5: **C07D 239/69**, A01N 47/36, C07D 403/12, C07D 251/46, C07D 251/16

(30) Priorität: **23.08.89 DE 3927770**

(43) Veröffentlichungstag der Anmeldung: **27.02.91 Patentblatt 91/09**

(84) Benannte Vertragsstaaten: **BE CH DE FR GB IT LI NL**

(71) Anmelder: **BAYER AG**

**D-5090 Leverkusen 1 Bayerwerk(DE)**

(72) Erfinder: **Fest, Christa, Dr.**
**Im Johannistal 20**
**D-5600 Wuppertal 1(DE)**
Erfinder: **Kirsten, Rolf, Dr.**
**Carl-Langhans-Strasse 27**
**D-4019 Monheim(DE)**
Erfinder: **Kluth, Joachim, Dr.**
**Tannenweg 9**
**D-4018 Langenfeld(DE)**
Erfinder: **Müller, Klaus-Helmut, Dr.**
**Bockhackstrasse 55**
**D-4000 Düsseldorf 13(DE)**
Erfinder: **Riebel, Hans-Jochem, Dr.**
**In der Beek 92**
**D-5600 Wuppertal 1(DE)**
Erfinder: **Lürssen, Klaus, Dr.**
**August-Kierspel-Strasse 145**
**D-5060 Bergisch Gladbach 2(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**

(54) **Substituierte Arylsulfonylaminoguanidinoazine.**

(57) Die Erfindung betrifft neue substituierte Arylsulfonylaminoguanidinoazine der Formel (I),

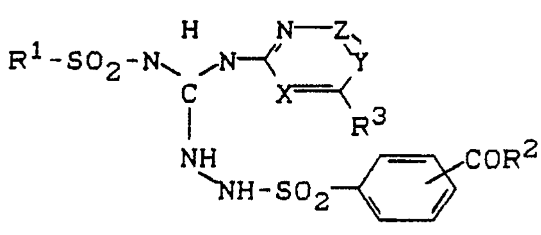

(I)

in welcher
$R^1$ für jeweils gegebenenfalls substituiertes Aryl, Aralkyl oder Heteroaryl steht,
$R^2$ für Hydroxy, Amino oder für jeweils gegebenenfalls substituiertes Alkoxy oder Alkylamino steht,
$R^3$ für Wasserstoff, Halogen, Hydroxy, Alkyl, Halogenalkyl, Alkoxyalkyl, Alkoxy, Halogenalkoxy, Alkylthio, Halogenalkylthio, Amino, Alkylamino oder Dialkylamino steht,
X für Stickstoff oder eine -CH-Gruppierung steht,
Y für Stickstoff oder eine $-CR^4$-Gruppierung steht, worin
$R^4$ für Wasserstoff, Halogen, Cyano, Alkyl, Formyl, Alkyl-carbonyl oder Alkoxy-carbonyl steht, und
Z für Stickstoff oder eine $-CR^5$-Gruppierung steht, worin

EP 0 414 067 A2

$R^5$ für Wasserstoff, Halogen, Hydroxy, Alkyl, Alkoxy, Alkylthio, Alkylamino oder Dialkylamino steht,
- ausgenommen die Verbindung N′-(4,6-Dimethyl-pyrimidin-2-yl)-N″-(2-methoxycarbonyl-phenylsulfonylamino)-N‴-(2-methoxycarbonyl-phenylsulfonyl)-guanidin, -

Verfahren zu ihrer Herstellung und ihre Verwendung als Herbizide.

## SUBSTITUIERTE ARYLSULFONYLAMINOGUANIDINOAZINE

Die Erfindung betrifft neue substituierte Arylsulfonylaminoguanidinoazine, Verfahren zu ihrer Herstellung und ihre Verwendung als Herbizide.

Es ist bereits bekannt, daß bestimmte Sulfonylaminoguanidinoazine, wie z.B. N′-(4,6-Dimethyl-pyrimidin-2-yl)-N″-(4-methyl-phenylsulfonylamino)-N‴-(2-chlor-phenylsulfonyl)-guanidin und N′-(4,6-Dimethyl-pyrimidin-2-yl)-N″-(2-methoxycarbonyl-phenylsulfonylamino)-N‴-(2-methoxycarbonyl-phenylsulfonyl)-guanidin, herbizide Eigenschaften aufweisen (vgl. EP-A 121082 und EP-A 302378). Die Herbizidwirkung dieser bekannten Verbindungen ist jedoch nicht in allen Belangen zufriedenstellend.

Es wurden nun neue substituierte Arylsulfonylaminoguanidinoazine der allgemeinen Formel (I)

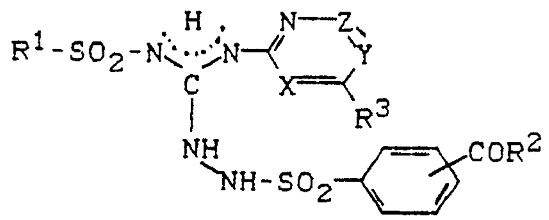

(I)

in welcher

$R^1$ für jeweils gegebenenfalls substituiertes Aryl, Aralkyl oder Heteroaryl steht,

$R^2$ für Hydroxy, Amino oder für jeweils gegebenenfalls substituiertes Alkoxy oder Alkylamino steht,

$R^3$ für Wasserstoff, Halogen, Hydroxy, Alkyl, Halogenalkyl, Alkoxyalkyl, Alkoxy, Halogenalkoxy, Alkylthio, Halogenalkylthio, Amino, Alkylamino oder Dialkylamino steht,

X für Stickstoff oder eine -CH-Gruppierung steht,

Y für Stickstoff oder eine $-CR^4$-Gruppierung steht, worin

$R^4$ für Wasserstoff, Halogen, Cyano, Alkyl, Formyl, Alkyl-carbonyl oder Alkoxy-carbonyl steht, und

Z für Stickstoff oder eine $-CR^5$-Gruppierung steht, worin

$R^5$ für Wasserstoff, Halogen, Hydroxy, Alkyl, Alkoxy, Alkylthio, Alkylamino oder Dialkylamino steht,

gefunden, wobei N′-(4,6-Dimethyl-pyrimidin-2-yl)-N″-(2-methoxycarbonyl-phenylsulfonylamino)-N‴-(2-methoxycarbonyl-phenylsulfonyl)-guanidin - bekannt aus EP-A 302378 - ausgenommen ist.

Die allgemeine Formel (I) steht für die einzelnen möglichen Tautomeren der Formeln (IA), (IB) und (IC)

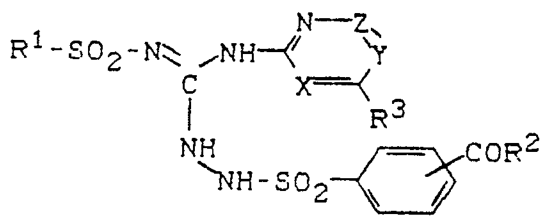

(IA)

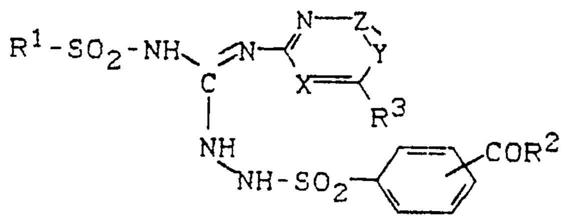

(IB)

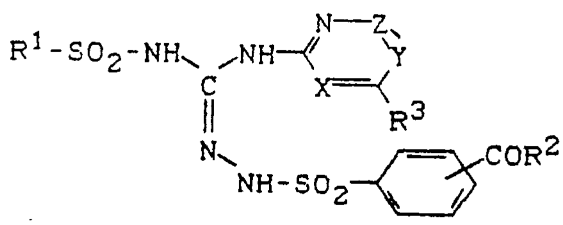

(IC)

sowie für Gemische dieser Tautomeren.

Man erhält die neuen substituierten Arylsulfonylaminoguanidinoazine der allgemeinen Formel (I), wenn man

(a) Sulfonylverbindungen der allgemeinen Formel (II)

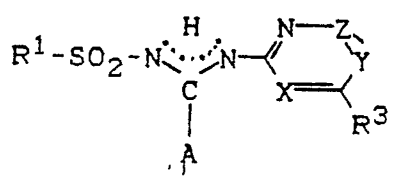

(II)

in welcher
$R^1$, $R^3$, X, Y und Z die oben angegebenen Bedeutungen haben und
A für eine der nachstehend angegebenen Abgangsgruppen

$R^6-SO_2-N-OR^7$ oder $-Q-R^8$ steht,

worin
$R^6$ die oben für $R^1$ angegebene Bedeutung hat, aber nicht in jedem Einzelfall mit $R^1$ identisch sein muß,
$R^7$ für Alkyl, Alkenyl oder Aralkyl steht,
$R^8$ für Alkyl, Aralkyl oder Aryl steht und
Q für Sauerstoff oder Schwefel steht,
mit Sulfonsäurehydraziden der allgemeinen Formel (III)

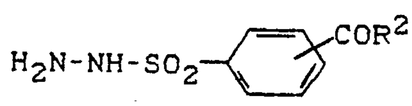

(III)

in welcher
$R^2$ die oben angegebene Bedeutung hat,

gegebenenfalls in Gegenwart eines Verdünnungsmittels umsetzt, oder wenn man
(b) Aminoguanidinoazine der allgemeinen Formel (IV)

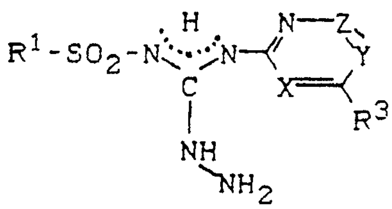
(IV)

in welcher
$R^1$, $R^3$, X, Y und Z die oben angegebenen Bedeutungen haben,
mit Sulfonsäurehalogeniden der allgemeinen Formel (V)

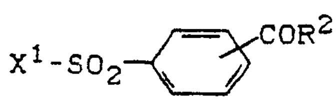
(V)

in welcher
$R^2$ die oben angegebene Bedeutung hat und
$X^1$ für Halogen steht,
oder mit Sulfobenzoesäureanhydrid der Formel (VI)

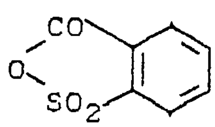
(VI)

gegebenenfalls in Gegenwart eines Verdünnungsmittels und gegebenenfalls in Gegenwart eines Säureakzeptors umsetzt, oder wenn man
(c) substituierte Arylsulfonylaminoguanidinoazine der allgemeinen Formel (I), in welcher $R^2$ für gegebenenfalls substituiertes Alkoxy steht und $R^1$, $R^3$, X, Y und Z die oben angegebenen Bedeutungen haben, mit wäßrigen Alkalilaugen oder mit Ammoniak oder mit gegebenenfalls substituierten Alkylaminen gegebenenfalls in Gegenwart eines Verdünnungsmittels umsetzt.

Die neuen substituierten Arylsulfonylaminoguanidinoazine der allgemeinen Formel (I) zeichnen sich durch starke herbizide Wirksamkeit aus.

Überraschenderweise zeigen die neuen Verbindungen der allgemeinen Formel (I) erheblich bessere Wirkung, insbesondere eine deutlich bessere Kulturpflanzen-Verträglichkeit, als die oben genannten vorbekannten Sulfonylaminoguanidinoazine, welche nach Struktur und Wirkprofil vergleichbare Stoffe sind.

Gegenstand der Erfindung sind vorzugsweise Verbindungen der Formel (I), in welcher
$R^1$ für den Rest

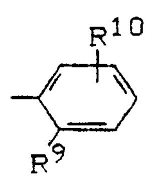

steht, worin
$R^9$ und $R^{10}$ gleich oder verschieden sind und für Wasserstoff, Fluor, Chlor, Brom, Iod, Cyano, Nitro, $C_1$-$C_6$-Alkyl (welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)-amino-carbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylaminocarbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist), für $C_2$-$C_6$-Alkenyl (welches gegebenenfalls durch Fluor, Chlor, Brom, Cya no, $C_1$-$C_4$-Alkoxy-carbonyl, Carboxy oder

Phenyl substituiert ist), für $C_2$-$C_6$-Alkinyl (welches gegebenenfalls durch Fluor, Chlor, Brom, Cyano, $C_1$-$C_4$-Alkoxy-carbonyl, Carboxy oder Phenyl substituiert ist), für $C_1$-$C_4$-Alkoxy (welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist), für $C_1$-$C_4$-Alkylthio (welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist), für $C_3$-$C_6$-Alkenyloxy (welches gegebenenfalls durch Fluor, Chlor, Brom, Cyano oder $C_1$-$C_4$-Alkoxy-carbonyl substituiert ist), für $C_2$-$C_6$-Alkenylthio (welches gegebenenfalls durch Fluor, Chlor, Brom, Cyano, Nitro, $C_1$-$C_3$-Alkylthio oder $C_1$-$C_4$-Alkoxycarbonyl substituiert ist), $C_3$-$C_6$-Alkinyloxy, $C_3$-$C_6$-Alkinylthio oder für den Rest $-S(O)_p-R^{11}$ stehen, wobei

p für die Zahlen 1 oder 2 steht und

$R^{11}$ für $C_1$-$C_4$-Alkyl (welches gegebenenfalls durch Fluor, Chlor, Brom, Cyano oder $C_1$-$C_4$-Alkoxy-carbonyl substituiert ist), $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest $-NHOR^{12}$ steht, wobei

$R^{12}$ für $C_1$-$C_{12}$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzylhydryl oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist) steht,

$R^9$ und $R^{10}$ weiterhin für Phenyl oder Phenoxy, für Amino, $C_1$-$C_4$-Alkylcarbonylamino, $C_1$-$C_4$-Alkoxy-carbonylamino, $C_1$-$C_4$-Alkylamino-carbonyl-amino, Di-($C_1$-$C_4$-alkyl)-amino-carbonyl-amino, oder für den Rest $-CO-R^{13}$ stehen, wobei

$R^{13}$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$-Alkylthio, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht (welche gegebenenfalls durch Fluor und/oder Chlor substituiert sind),

$R^9$ und $R^{10}$ weiterhin für $C_1$-$C_4$-Alkylsulfonyloxy, Di-($C_1$-$C_4$-alkyl)-aminosulfonylamino, Thiazolyloxy oder für den Rest $-CH=N-R^{14}$ stehen, wobei

$R^{14}$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht, weiterhin

$R^1$ für den Rest

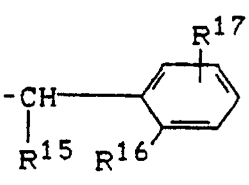

steht, worin

$R^{15}$ für Wasserstoff oder $C_1$-$C_4$-Alkyl steht,

$R^{16}$ und $R^{17}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1$-$C_4$-Alkoxy (welches gegebenenfalls durch Fluor und/ oder Chlor substituiert ist), Carboxy, $C_1$-$C_4$-Alkoxy-carbonyl, $C_1$-$C_4$-Alkylsulfonyl oder Di-($C_1$-$C_4$-alkyl)-aminosulfonyl stehen; weiterhin

$R^1$ für den Rest

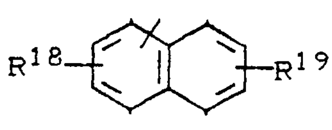

steht, worin

$R^{18}$ und $R^{19}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist) oder $C_1$-$C_4$-Alkoxy (welches gegebenenfalls durch Fluor und/oder Chlor substituiert ist), stehen; weiterhin

$R^1$ für den Rest

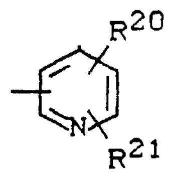

steht, worin

$R^{20}$ und $R^{21}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/ oder Chlor substituiert sind), sowie für Di-($C_1$-$C_4$-alkyl)-aminocarbonyl oder $C_1$-$C_4$-Alkoxy-carbonyl stehen; weiterhin

$R^1$ für den Rest

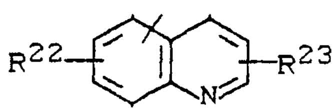

steht, worin

$R^{22}$ und $R^{23}$ gleich oder verschieden sind und für Wasserstoff, Fluor, Chlor, Brom, $C_1$-$C_4$-Alkyl (welches gegebenenfalls durch Fluor und/oder Brom substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), oder für Di-($C_1$-$C_4$-alkyl)-aminosulfonyl stehen; weiterhin

$R^1$ für den Rest

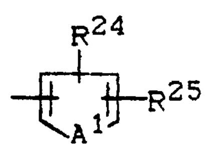

steht, worin

$R^{24}$ und $R^{25}$ gleich oder verschieden sind und für Wasserstoff, Fluor, Chlor, Brom, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), $C_1$-$C_4$-Alkylthio, $C_1$-$C_4$-Alkylsulfinyl oder $C_1$-$C_4$-Alkylsulfonyl (welches gegebenenfalls durch Fluor und/oder Chlor substituiert ist), Di-($C_1$-$C_4$-alkyl)-aminosulfonyl oder $C_1$-$C_4$-Alkoxycarbonyl stehen, und

$A^1$ für Sauerstoff, Schwefel oder die Gruppierung N-$Z^1$ steht, wobei

$Z^1$ für Wasserstoff, $C_1$-$C_4$-Alkyl (welches gegebenenfalls durch Fluor, Chlor, Brom oder Cyano substituiert ist), $C_3$-$C_6$-Cycloalkyl, Benzyl, Phenyl (welches gegebenenfalls durch Fluor, Chlor, Brom oder Nitro substituiert ist), $C_1$-$C_4$-Alkylcarbonyl, $C_1$-$C_4$-Alkoxy-carbonyl oder Di-($C_1$-$C_4$-alkyl)-aminocarbonyl steht; weiterhin

$R^1$ für den Rest

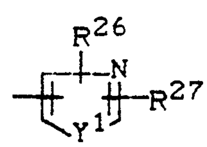

steht, worin
$R^{26}$ für Wasserstoff, $C_1$-$C_5$-Alkyl oder Halogen steht,
$R^{27}$ für Wasserstoff oder $C_1$-$C_5$-Alkyl steht und
$Y^1$ für Schwefel oder die Gruppierung N-$R^{28}$ steht, wobei
$R^{28}$ für Wasserstoff oder $C_1$-$C_5$-Alkyl steht, weiterhin
$R^1$ für den Rest

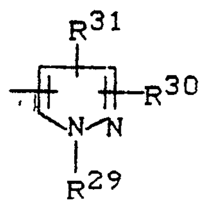

steht, worin
$R^{29}$ für Wasserstoff, $C_1$-$C_4$-Alkyl, Phenyl, Naphthyl oder (Iso)chinolinyl steht,
$R^{30}$ für Wasserstoff, Halogen, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist) oder $C_1$-$C_4$-Alkoxycarbonyl steht und
$R^{31}$ für Wasserstoff, Halogen oder $C_1$-$C_4$-Alkyl steht,
weiterhin
$R^1$ für den Rest

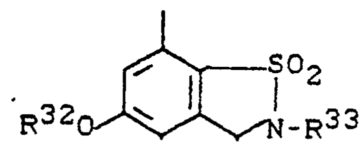

steht, worin
$R^{32}$ für $C_1$-$C_3$-Alkyl steht und
$R^{33}$ für $C_1$-$C_4$-Alkyl steht,
weiterhin
$R^1$ für den Rest

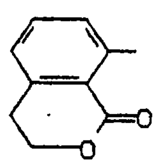

steht,
weiterhin
$R^2$ für Hydroxy, Amino oder für jeweils gegebenenfalls durch Fluor, Chlor, Methoxy oder Ethoxy substituiertes $C_1$-$C_4$-Alkoxy oder $C_1$-$C_4$-Alkylamino steht,
weiterhin
$R^3$ für Wasserstoff, Fluor, Chlor, Brom, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino, Dimethylamino oder Diethylamino steht,
X für Stickstoff oder eine -CH-Gruppierung steht,
Y für Stickstoff oder eine -$CR^4$-Gruppierung steht, worin
$R^4$ für Wasserstoff, Fluor, Chlor, Brom, Cyano, Methyl, Formyl, Acetyl, Methoxycarbonyl oder Ethoxycarbonyl steht, und
Z für Stickstoff oder eine -$CR^5$-Gruppierung steht, worin
$R^5$ für Wasserstoff, Fluor, Chlor, Brom, $C_1$-$C_4$-Alkyl, $C_1$-$C_4$-Alkoxy, $C_1$-$C_4$-Alkylthio, $C_1$-$C_4$-Alkylamino, Dimethylamino oder Diethylamino steht,

wobei N′-(4,6-Dimethyl-pyrimidin-2-yl)-N″-(2-methoxycarbonyl-phenylsulfonylamino)-N‴-(2-methoxycarbonylphenylsulfonyl)-guanidin ausgenommen ist.

Gegenstand der Erfindung sind insbesondere Verbindungen der Formel (I), in welcher

$R^1$ für den Rest

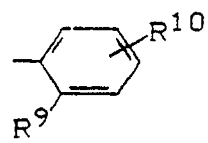

steht worin

$R^9$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, Diethylaminosulfonyl, N-Methoxy-N-methylaminosulfonyl, Phenyl, Phenoxy oder $C_1$-$C_3$-Alkoxycarbonyl steht und

$R^{10}$ für Wasserstoff steht; weiterhin

$R^1$ für den Rest

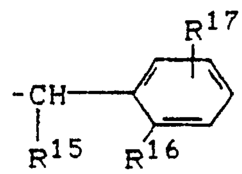

steht, worin

$R^{15}$ für Wasserstoff steht,

$R^{16}$ für Fluor, Chlor, Brom, Methyl, Methoxy, Ethoxy, Difluormethoxy, Trifluormethoxy, Methoxycarbonyl, Ethoxycarbonyl, Methylsulfonyl oder Dimethylaminosulfonyl steht und

$R^{17}$ für Wasserstoff steht; weiterhin

$R^1$ für den Rest

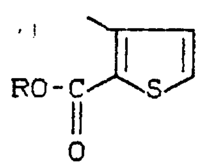

steht, worin

R für Methyl oder Ethyl steht, oder

$R^1$ für den Rest

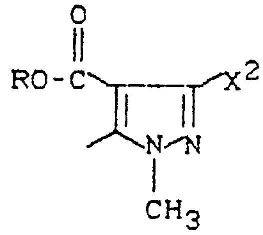

steht, worin

R für Methyl oder Ethyl steht und

$X^2$ für Wasserstoff oder Chlor steht, weiterhin

$R^2$ für Hydroxy, Amino, Methoxy, Ethoxy, Propoxy, Isopropoxy, 2-Chlor-ethoxy, 2-Methoxy-ethoxy, 2-Ethoxy-ethoxy, Methylamino, Ethylamino, Propylamino oder Isopropylamino steht,

weiterhin

$R^3$ für Wasserstoff, Fluor, Chlor, Brom, Methyl, Trifluormethyl, Methoxy, Ethoxy, Difluormethoxy, Methylthio, Ethylthio, Amino, Methylamino, Ethylamino, Dimethylamino oder Diethylamino steht,
X für Stickstoff oder eine -CH-Gruppierung steht,
Y für Stickstoff oder eine -$CR^4$-Gruppierung steht, worin
$R^4$ für Wasserstoff, Fluor, Chlor oder Methyl steht, und
Z für Stickstoff oder eine -$CR^5$-Gruppierung steht, worin
$R^5$ für Wasserstoff, Fluor, Chlor, Brom, Methyl, Ethyl, Methoxy, Ethoxy, Propoxy, Isopropoxy, Methylthio, Ethylthio, Methylamino, Ethylamino, Dimethylamino oder Diethylamino steht,
wobei $N'$-(4,6-Dimethyl-pyrimidin-2-yl)-$N''$-(2-methoxycarbonyl-phenylsulfonylamino)-$N'''$-(2-methoxycarbonylphenylsulfonyl)-guanidin ausgenommen ist.

Ganz besonders bevorzugt sind diejenigen Verbindungen der Formel (I) bei welchen die Gruppierung -$COR^2$ sich in ortho-Position zur $SO_2$-Gruppe befindet und $R^1$, $R^2$, $R^3$, X, Y und Z die oben als insbesondere bevorzugt angegebenen Bedeutungen haben.

Verwendet man beispielsweise $N'$-(4,6-Dimethoxy-s-triazin-2-yl)-$N''$-methoxy-$N''$,$N'''$-bis-(2-brom-phenylsulfonyl)-guanidin und 2-Methoxycarbonyl-benzolsulfonsäurehydrazid als Ausgangsstoffe, so kann der Reaktionsablauf beim erfindungsgemäßen Verfahren (a) durch das folgende Formelschema skiziert werden:

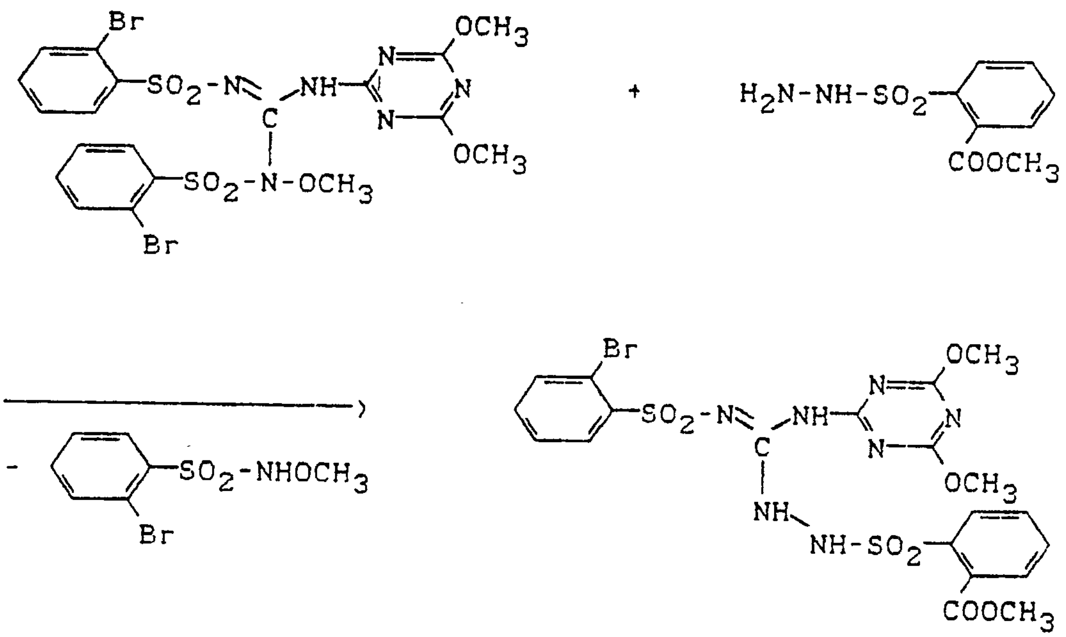

Verwendet man beispielsweise $N'$-(4,6-Dimethoxy-pyrimidin-2-yl)-$N''$-amino-$N'''$-(2-difluormethoxy-phenylsulfonyl)-guanidin und 2-Ethoxycarbonylbenzolsulfonsäurechlorid als Ausgangsstoffe, so kann der Reaktionsablauf beim erfindungsgemäßen Verfahren (b) durch das folgende Formelschema skizziert werden:

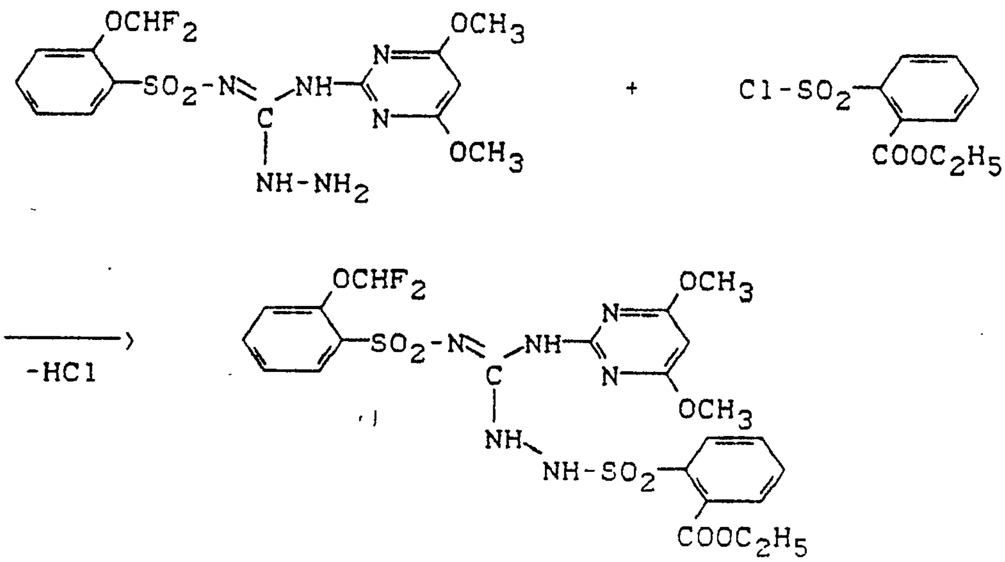


Verwendet man beispielsweise N′-(4-Methoxy-6-methyl-pyrimidin-2-yl)-N″-(2-methoxycarbonyl-phenylsulfonylamino)-N‴-(2-methoxycarbonyl-phenylsulfonyl)-guanidin und Ammoniak als Ausgangsstoffe, so kann der Reaktionsablauf beim erfindungsgemäßen Verfahren (c) durch das folgende Formelschema skizziert werden:

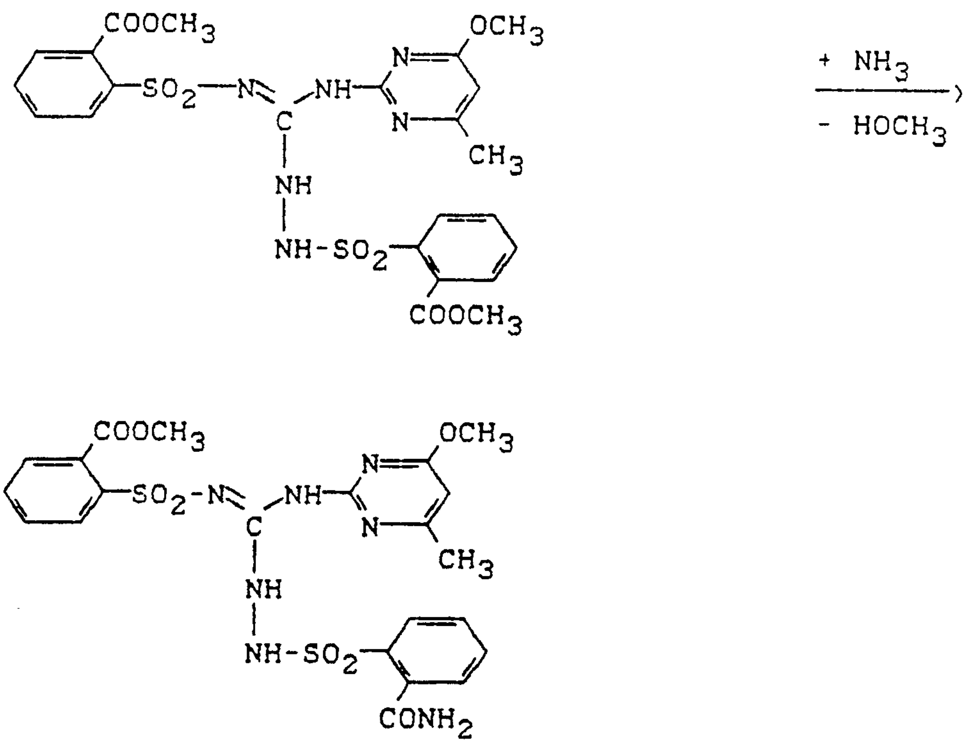


Die beim erfindungsgemäßen Verfahren (a) als Ausgangsstoffe zu verwendenden Sulfonylverbindungen sind durch die Formel (II) allgemein definiert. In Formel (II) haben $R^1$, $R^3$, X, Y und Z vorzugsweise bzw. insbesondere diejenigen Bedeutungen, die bereits oben im Rahmen der Beschreibung der erfindungsgemäßen Verbindungen der Formel (I) als bevorzugt bzw. als insbesondere bevorzugt für $R^1$, $R^3$, X, Y und Z angegeben wurden und

A steht vorzugsweise für eine der nachstehend angegebenen Abgangsgruppen

$R^6-SO_2-N-OR^7$ oder $-Q-R^8$, worin

$R^6$ die oben für $R^1$ vorzugsweise angegebene Bedeutung hat, aber nicht in jedem Einzelfall mit $R^1$ identisch sein muß,
$R^7$ für $C_1$-$C_4$-Alkyl, $C_3$-$C_4$-Alkenyl oder Benzyl steht,
$R^8$ für $C_1$-$C_4$-Alkyl, Benzyl oder Phenyl steht und
Q für Sauerstoff oder Schwefel steht.
Insbesondere steht A für die Gruppierung

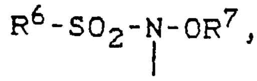

worin
$R^6$ die oben für $R^1$ als insbesondere bevorzugt angegebene Bedeutung hat, aber nicht in jedem Einzelfall mit $R^1$ identisch sein muß und
$R^7$ für Methyl steht,
oder A steht für die Gruppierung
-Q-$R^8$, worin
$R^8$ für Methyl oder Phenyl steht und
Q für Sauerstoff oder Schwefel steht.

Beispiele für die Ausgangsstoffe der Formel (II) sind in der nachstehenden Tabelle 1 aufgeführt.

Tabelle 1: Beispiele für die Ausgangsstoffe der Formel (II)

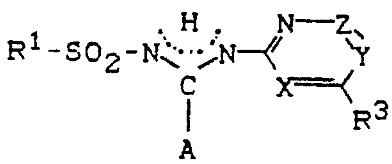

(II)

<u>Tabelle 1</u>

| A | $R^1$ | $R^3$ | X | Y | Z |
|---|---|---|---|---|---|
| 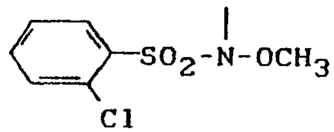 | 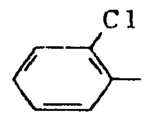 | $CH_3$ | N | CH | C-$OCH_3$ |
| 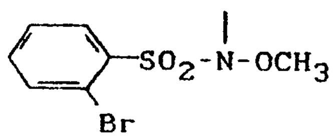 | 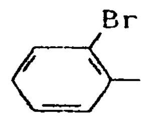 | $OCH_3$ | N | CH | C-$OCH_3$ |
| 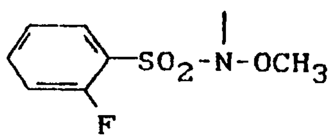 | 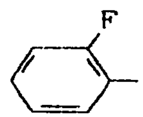 | $OCH_3$ | N | CH | C-$OCH_3$ |
| 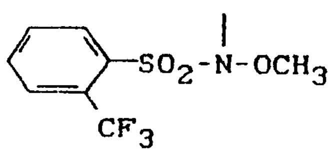 | 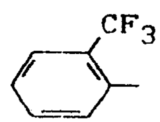 | $OCH_3$ | N | CH | C-$OCH_3$ |
| 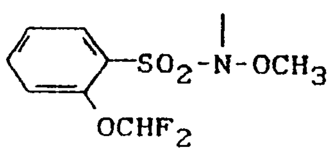 | 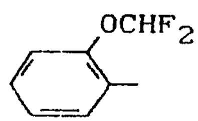 | $OCH_3$ | N | CH | C-$OCH_3$ |

Tabelle 1 - Fortsetzung

| A | $R^1$ | $R^3$ | X | Y | Z |
|---|---|---|---|---|---|
| 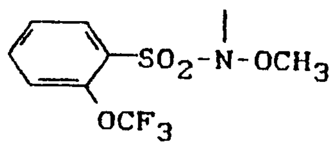 | 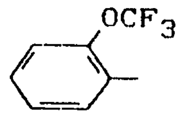 | $OCH_3$ | N | CH | $C-OCH_3$ |
| 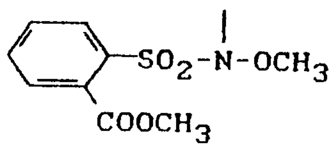 | 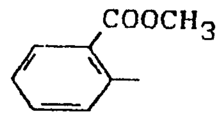 | $CH_3$ | N | CH | $C-CH_3$ |
| 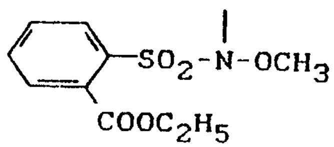 | 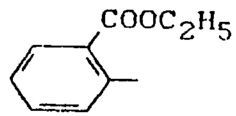 | $CH_3$ | N | CH | $C-CH_3$ |
| 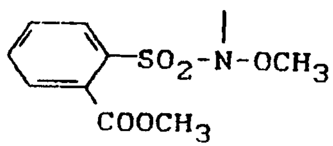 | 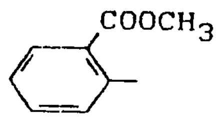 | $OCH_3$ | N | CH | $C-OCH_3$ |
| 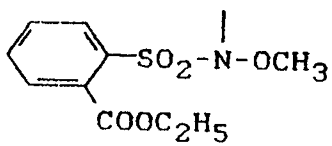 | 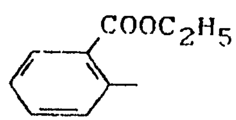 | $OCH_3$ | N | CH | $C-OCH_3$ |

Tabelle 1 - Fortsetzung

| A | $R^1$ | $R^3$ | X | Y | Z |
|---|---|---|---|---|---|
| 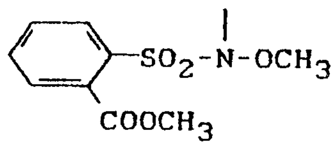 | 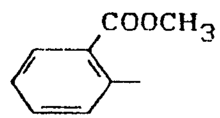 | $C_2H_5$ | N | CH | C-$OCH_3$ |
| 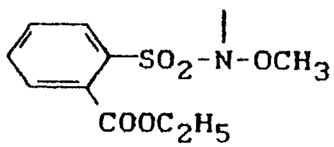 | 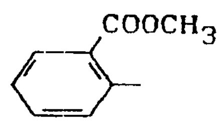 | $C_2H_5$ | N | CH | C-$OCH_3$ |
| 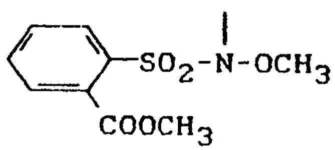 | 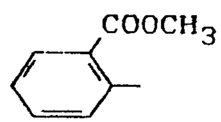 | $OCH_3$ | N | CH | C-Cl |
| 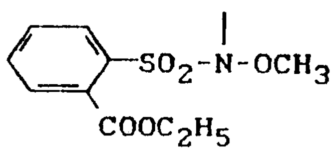 | 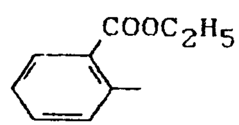 | $OCH_3$ | N | CH | C-Cl |
| 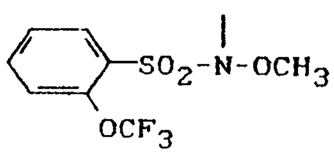 | 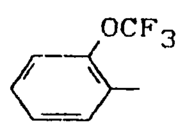 | $OCH_3$ | N | CH | C-Cl |

**Tabelle 1 - Fortsetzung**

| A | $R^1$ | $R^3$ | X | Y | Z |
|---|---|---|---|---|---|
| 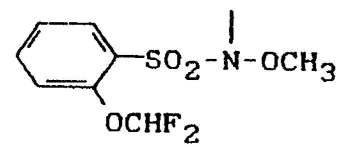 | 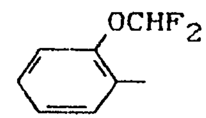 | $OCH_3$ | N | CH | C-Cl |
| 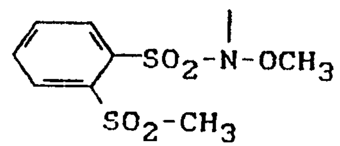 | 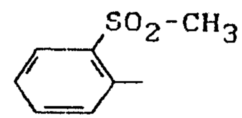 | H | N | CH | $C-CH_3$ |
| 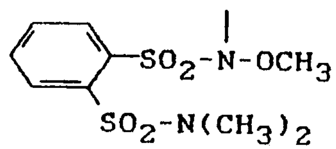 | 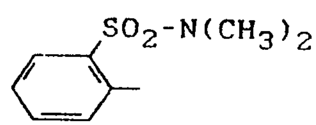 | $CH_3$ | N | CH | $C-OCH_3$ |
| 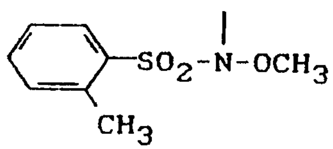 | 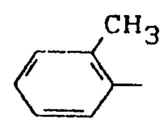 | $OCH_3$ | N | CH | $C-OCH_3$ |
| 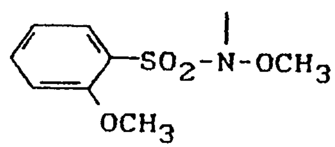 | 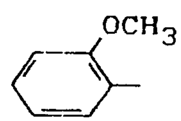 | $CH_3$ | N | CH | $C-OCH_3$ |

Tabelle 1 - Fortsetzung

| A | $R^1$ | $R^3$ | X | Y | Z |
|---|---|---|---|---|---|
| 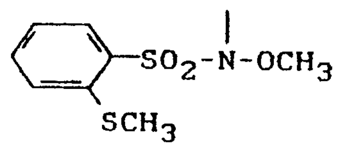 | 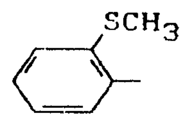 | $OCH_3$ | N | CH | $C-OCH_3$ |
| 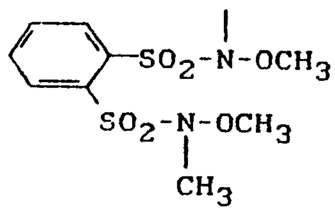 | 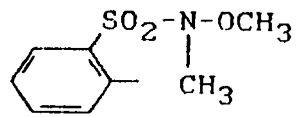 | $OCH_3$ | N | CH | $C-OCH_3$ |
| 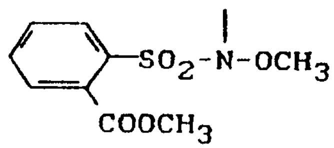 | 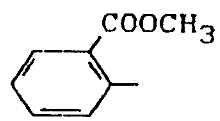 | $CH_3$ | N | CH | $C-OC_2H_5$ |
| 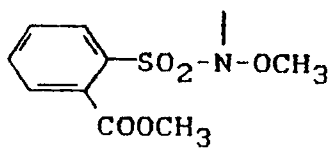 | 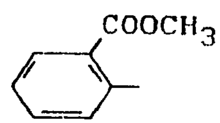 | $OCHF_2$ | N | CH | $C-CH_3$ |

<u>Tabelle 1</u> - Fortsetzung

| A | $R^1$ | $R^3$ | X | Y | Z |
|---|---|---|---|---|---|
| 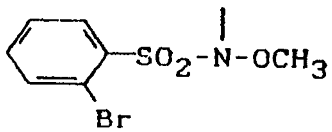 | 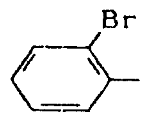 | $CH_3$ | N | CH | $C-SCH_3$ |
| 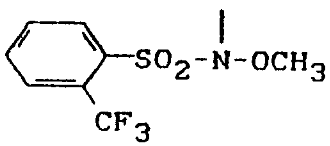 | 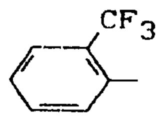 | $CH_3$ | N | CH | $C-N(CH_3)_2$ |
| 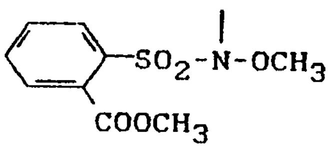 | 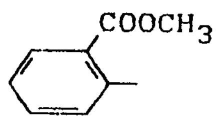 | $OCHF_2$ | N | CH | $C-OCHF_2$ |
| 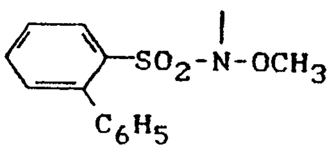 | 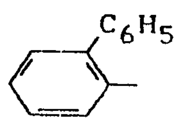 | $OCH_3$ | N | CH | $C-OCH_3$ |
| 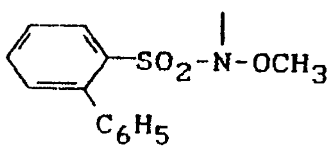 | 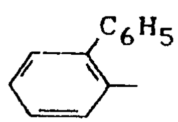 | $OCH_3$ | N | N | $C-OCH_3$ |

Tabelle 1 - Fortsetzung

| A | $R^1$ | $R^3$ | X | Y | Z |
|---|---|---|---|---|---|
| 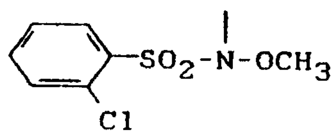 | 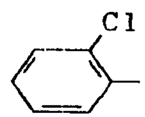 | $CH_3$ | N | N | $C-OCH_3$ |
| 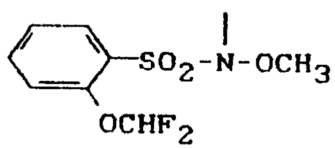 | 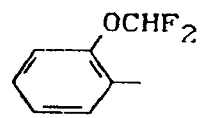 | $OCH_3$ | N | N | $C-OCH_3$ |
| 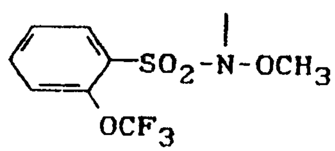 | 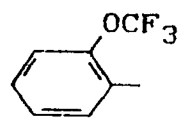 | $OCH_3$ | N | N | $C-OCH_3$ |
| 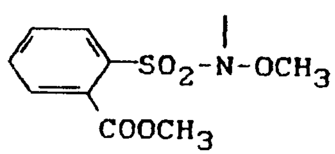 | 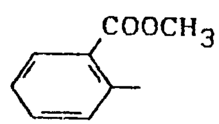 | $CH_3$ | N | N | $C-OCH_3$ |
| 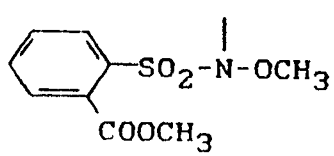 | 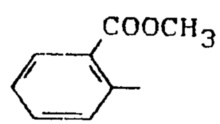 | $OCH_3$ | N | N | $C-OCH_3$ |

<u>Tabelle 1</u> - Fortsetzung

| A | $R^1$ | $R^3$ | X | Y | Z |
|---|---|---|---|---|---|
| 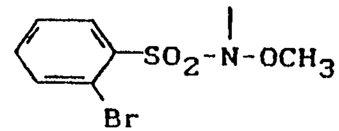 | 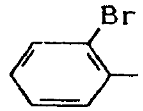 | $CH_3$ | N | N | $C-CH_3$ |
| 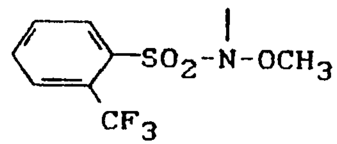 | 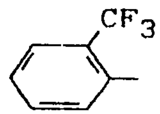 | $CH_3$ | N | N | C-Cl |
| 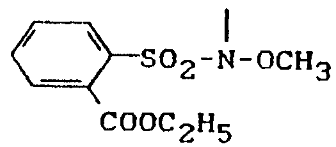 | 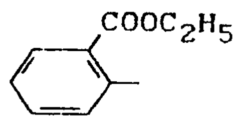 | $OCH_3$ | N | N | $C-OCH_3$ |
| 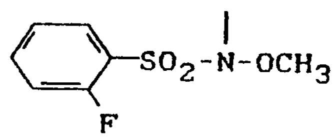 | 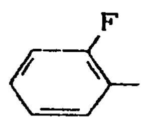 | $OCH_3$ | N | N | $C-OCH_3$ |
| 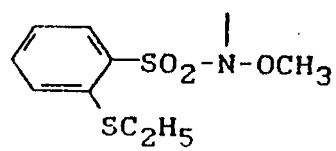 | 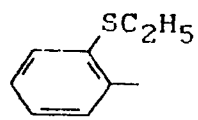 | $OCH_3$ | N | N | $C-OCH_3$ |

Tabelle 1 - Fortsetzung

| A | $R^1$ | $R^3$ | X | Y | Z |
|---|---|---|---|---|---|
| 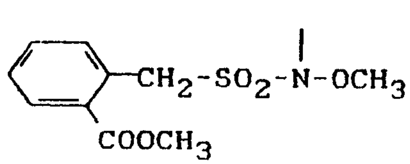 | 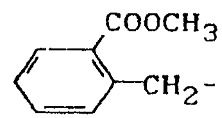 | $OCH_3$ | N | CH | $C-OCH_3$ |
| 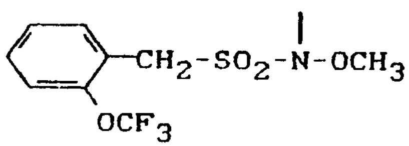 | 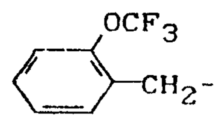 | $OCH_3$ | N | CH | $C-OCH_3$ |
| $-OC_6H_5$ | 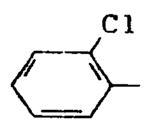 | $CH_3$ | N | CH | $C-CH_3$ |
| $-OCH_3$ | 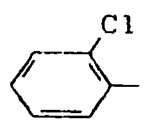 | $CH_3$ | N | CH | $C-OCH_3$ |
| $-SCH_3$ | 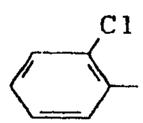 | $OCH_3$ | N | CH | $C-OCH_3$ |

Tabelle 1 - Fortsetzung

| A | $R^1$ | $R^3$ | X | Y | Z |
|---|---|---|---|---|---|
| $-SC_6H_5$ | 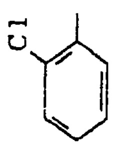 | $OCH_3$ | N | CH | $C-OCH_3$ |
| 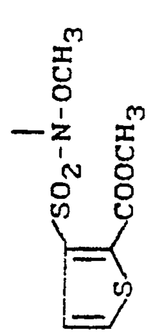 | 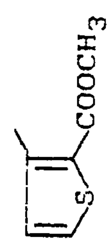 | $CH_3$ | N | N | $C-OCH_3$ |
| 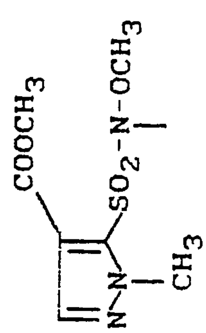 | 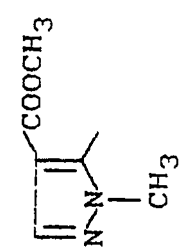 | $OCH_3$ | N | CH | $C-OCH_3$ |
| 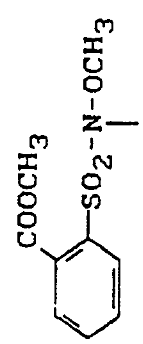 | 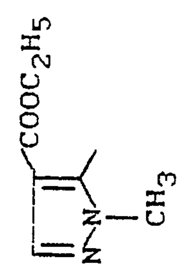 | $OCH_3$ | N | CH | $C-OCH_3$ |

Die Ausgangsstoffe der Formel (II) sind bekannt und/oder können nach an sich bekannten Verfahren hergestellt werden (vgl. EP-A 121 082, EP-A 172 957, EP-A 173 321, EP-A 173 956, EP-A 224 078, EP-A 5 986 und EP-A 24 215).

Die beim erfindungsgemäßen Verfahren weiter als Ausgangsstoffe zu verwendenden Sulfonsäurehydrazide sind durch die Formel (III) allgemein definiert. In Formel (III) hat $R^2$ vorzugsweise bzw. insbesondere diejenige Bedeutung, die bereits oben im Rahmen der Beschreibung der erfindungsgemäßen Verbindungen der Formel (I) als bevorzugt bzw. als insbesondere bevorzugt für $R^2$ angegeben wurde.

Als Beispiele für die Verbindungen der Formel (III) seien genannt:
2-Methoxycarbonyl-, 4-Methoxycarbonyl-, 2-Ethoxycarbonyl- und 4-Ethoxycarbonyl-benzolsulfonsäurehydrazid.

Die Ausgangsstoffe der Formel (III) sind bekannt und/oder können nach an sich bekannten Verfahren hergestellt werden (vgl. Org. Synth. 40 (1960), 93 - 95; EP-A 302378; Egypt. J. Pharm. Sci 22(1981), 207-221 - zit. in Chem. Abstracts 100, 191704y).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei vorzugsweise Wasser und/oder polare organische Lösungsmittel, wie Methanol, Ethanol, Isopropanol, Butanol, Isobutanol, sec-Butanol, tert-Butanol, Glycoldimethylether, Diglycoldimethyl ether, Tetrahydrofuran, Dioxan, Essigsäuremethylester, Essigsäureethylester, Acetonitril, Propionitril, Dimethylformamid, Dimethylacetamid, N-Methylpyrrolidon, Dimethylsulfoxid und Tetramethylensulfon, in Betracht.

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 100 °C.

Zur Durchführung des erfindungsgemäßen Verfahrens (a) setzt man je Mol Sulfonylverbindung der Formel (II) im allgemeinen zwischen 1 und 5 Mol, vorzugsweise zwischen 1 und 3 Mol, Sulfonsäurehydrazid der Formel (III) ein.

Im allgemeinen werden die Reaktionskomponenten bei Raumtemperatur oder unter Eiskühlung zusammen gegeben und das Reaktionsgemisch wird, gegebenenfalls bei erhöhter Temperatur, bis zum Ende der Umsetzung gerührt. Die Produkte der Formel (I) fallen im allgemeinen nach dem Abkühlen kristallin an und können durch Absaugen isoliert werden.

Die beim erfindungsgemäßen Verfahren (b) zur Herstellung von Verbindungen der Formel (I) als Ausgangsstoffe zu verwendenden Aminoguanidinoazine sind durch die Formel (IV) allgemein definiert.

In Formel (IV) haben $R^1$, $R^3$, X, Y und Z vorzugsweise bzw. insbesondere diejenigen Bedeutungen, die bereits oben im Zusammenhang mit der Beschreibung der erfin dungsgemäßen Verbindungen der Formel (I) vorzugsweise bzw. als insbesondere bevorzugt für $R^1$, $R^3$, X, Y und Z angegeben wurden.

Beispiele für die Ausgangsstoffe der Formel (IV) sind in der nachstehenden Tabelle 2 aufgeführt.

Tabelle 2: Beispiele für die Ausgangsstoffe der Formel (IV)

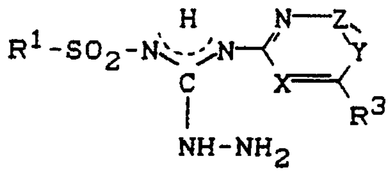

(IV)

| $R^1$ | $R^3$ | X | Y | Z |
|---|---|---|---|---|
| 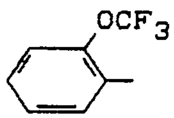 | $OCH_3$ | N | CH | $C-OCH_3$ |
| 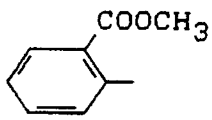 | $OCH_3$ | N | CH | $C-OCH_3$ |
| 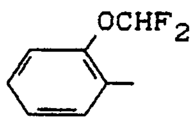 | $CH_3$ | N | CH | $C-CH_3$ |
| 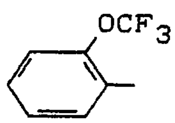 | $CH_3$ | N | N | $C-OCH_3$ |
| 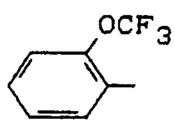 | $OCH_3$ | N | N | $C-OCH_3$ |
| 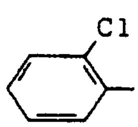 | $CH_3$ | N | CH | $C-OCH_3$ |
| 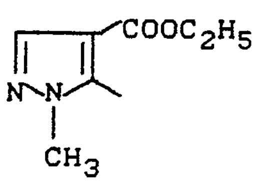 | $CH_3$ | N | CH | $C-CH_3$ |

Tabelle 2 - Fortsetzung

| $R^1$ | $R^3$ | X | Y | Z |
|---|---|---|---|---|
| 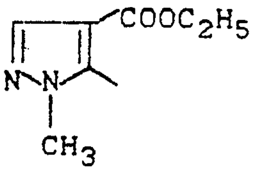 | $CH_3$ | N | N | $C-CH_3$ |
| 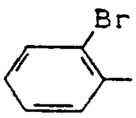 | $CH_3$ | N | CH | $C-CH_3$ |
| 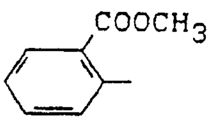 | $CH_3$ | N | N | $C-OCH_3$ |
| 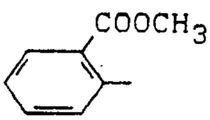 | $OCH_3$ | N | CH | $C-OCH_3$ |
| 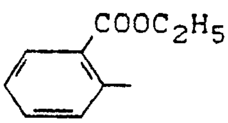 | $CH_3$ | N | CH | $C-OCH_3$ |
| 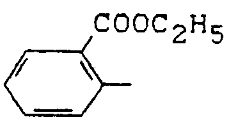 | $OCH_3$ | N | CH | $C-OCH_3$ |
| 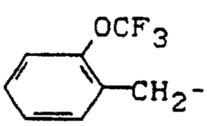 | $OCH_3$ | N | CH | $C-OCH_3$ |
| 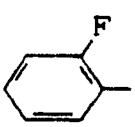 | $OCH_3$ | N | N | $C-OCH_3$ |

Tabelle 2 - Fortsetzung

| $R^1$ | $R^3$ | X | Y | Z |
|---|---|---|---|---|
| 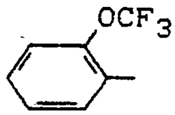 | $C_2H_5$ | N | CH | $C-OCH_3$ |
| 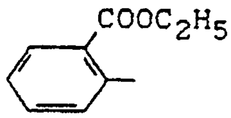 | $CH_3$ | N | N | $C-OCH_3$ |
| 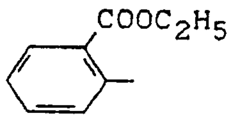 | $OCH_3$ | N | N | $C-OCH_3$ |
| 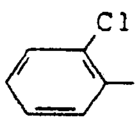 | $CH_3$ | N | N | $C-OCH_3$ |
| 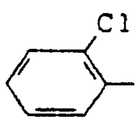 | $OCH_3$ | N | N | $C-OCH_3$ |
| 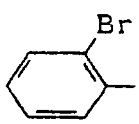 | $OCH_3$ | N | N | $C-OCH_3$ |
| 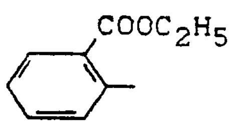 | $CH_3$ | N | CH | $C-CH_3$ |
| 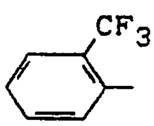 | $CH_3$ | N | CH | $C-CH_3$ |

Tabelle 2 - Fortsetzung

| $R^1$ | $R^3$ | X | Y | Z |
|---|---|---|---|---|
| 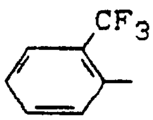 | $OCH_3$ | N | CH | $C-OCH_3$ |
| 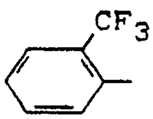 | $CH_3$ | N | N | $C-OCH_3$ |
| 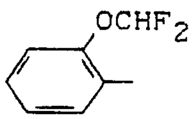 | $OCH_3$ | N | CH | $C-OCH_3$ |
| 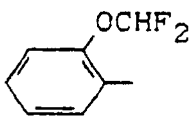 | $OCH_3$ | N | N | $C-OCH_3$ |
| 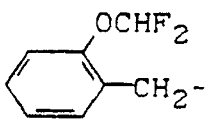 | $OCH_3$ | N | CH | $C-OCH_3$ |
| 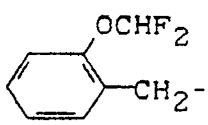 | $OCH_3$ | N | N | $C-OCH_3$ |
| 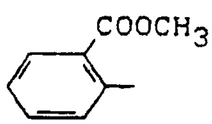 | $C_2H_5$ | N | N | $C-OCH_3$ |
| 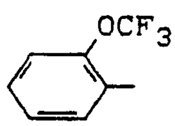 | $CH_3$ | N | CH | $C-OC_2H_5$ |

Tabelle 2 - Fortsetzung

| $R^1$ | $R^3$ | X | Y | Z |
|---|---|---|---|---|
| 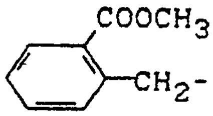 | $OCH_3$ | N | N | $C-OCH_3$ |
| 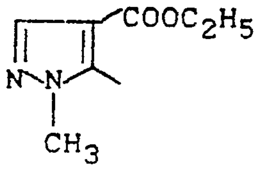 | $OCH_3$ | N | CH | $C-OCH_3$ |
| 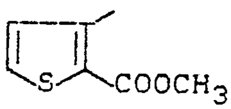 | $CH_3$ | N | N | $C-OCH_3$ |
| 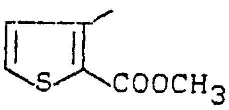 | $OCH_3$ | N | N | $C-OCH_3$ |
| 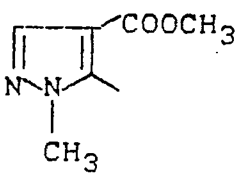 | $OCH_3$ | N | N | $C-OCH_3$ |
| 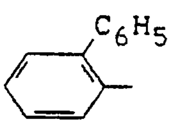 | $OCH_3$ | N | CH | $C-OCH_3$ |
| 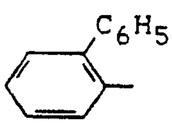 | $OCH_3$ | N | N | $C-OCH_3$ |
| 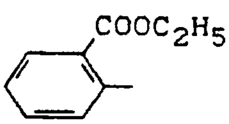 | $OCH_3$ | N | CH | C-Cl |

Tabelle 2 - Fortsetzung

| $R^1$ | $R^3$ | X | Y | Z |
|---|---|---|---|---|
| 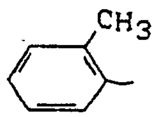 | $CH_3$ | N | N | $C-OCH_3$ |
| 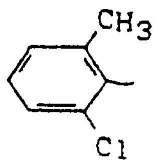 | $CH_3$ | N | CH | $C-CH_3$ |
| 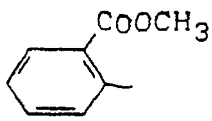 | $CF_3$ | N | CH | $C-OCH_3$ |
| 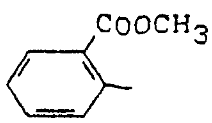 | $CH_3$ | N | CH | $C-OCHF_2$ |
| 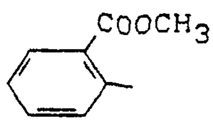 | $OCH_3$ | N | CH | $C-OCHF_2$ |
| 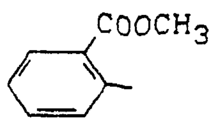 | $OCHF_2$ | N | CH | $C-OCHF_2$ |
| 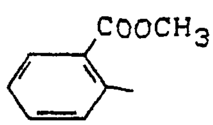 | $NHCH_3$ | N | N | $C-OC_2H_5$ |
| 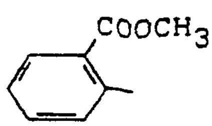 | $NHC_2H_5$ | N | N | $C-OCH_3$ |

Tabelle 2 - Fortsetzung

| $R^1$ | $R^2$ | X | Y | Z |
|---|---|---|---|---|
| 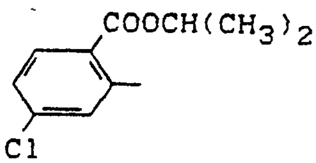 | $CH_3$ | N | N | $C-OCH_3$ |
| 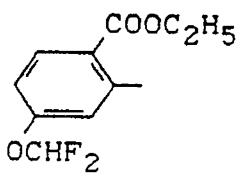 | $OCH_3$ | N | CH | $C-OCH_3$ |
| 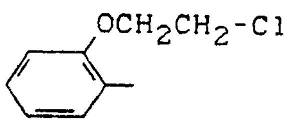 | $CH_3$ | N | N | $C-OCH_3$ |
| 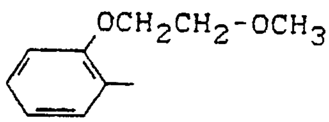 | $OCH_3$ | N | N | $C-OCH_3$ |
| 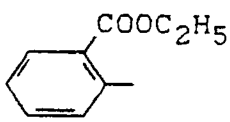 | Cl | N | CH | $C-OCH_3$ |
| 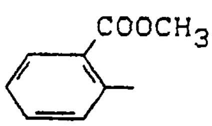 | $CH(OCH_3)_2$ | N | CH | $C-OCH_3$ |
| 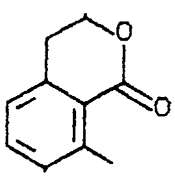 | $NHCH_3$ | N | N | $C-OC_2H_5$ |
| 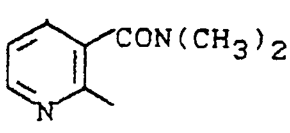 | $OCH_3$ | N | CH | $C-OCH_3$ |

Tabelle 2 - Fortsetzung

| $R^1$ | $R^3$ | X | Y | Z |
|---|---|---|---|---|
| 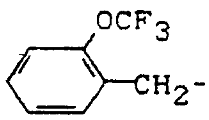 | $OCH_3$ | N | N | $C-OCH_3$ |
| 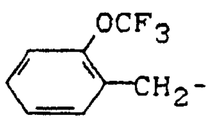 | $CH_3$ | N | N | $C-OCH_3$ |
| 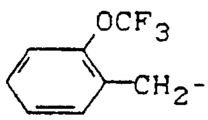 | $CH_3$ | N | N | $C-CH_3$ |
| 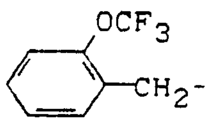 | $NHCH_3$ | N | N | $C-OC_2H_5$ |
| 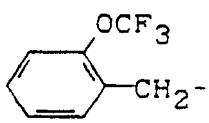 | $C_2H_5$ | N | N | $C-OCH_3$ |
| 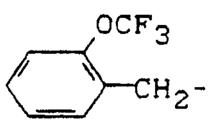 | $CH_3$ | N | N | $C-OC_2H_5$ |
| 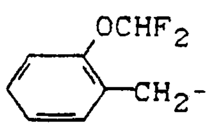 | $CH_3$ | N | N | $C-OCH_3$ |
| 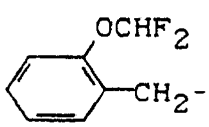 | $CH_3$ | N | N | $C-CH_3$ |

Tabelle 2 - Fortsetzung

| $R^1$ | $R^3$ | X | Y | Z |
|---|---|---|---|---|
| 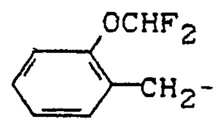 | $NHCH_3$ | N | N | $C-OC_2H_5$ |
| 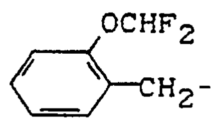 | $C_2H_5$ | N | N | $C-OCH_3$ |
| 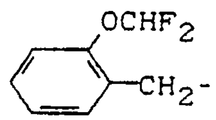 | $CH_3$ | N | N | $C-OC_2H_5$ |
| 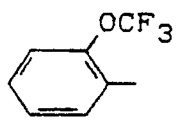 | $CF_3$ | N | CH | $C-OCH_3$ |
| 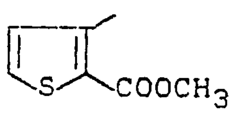 | $OCHF_2$ | N | CH | $C-OCHF_2$ |
| 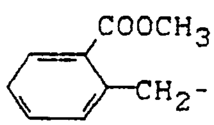 | $OCH_3$ | N | CH | $C-OCH_3$ |
| 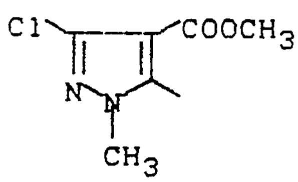 | $OCH_3$ | N | CH | $C-OCH_3$ |

Die Ausgangsstoffe der Formel (IV) sind bekannt und/oder können nach an sich bekannten Verfahren hergestellt werden (vgl. EP-A 224078, US-P 4725303, DE-Patentanmeldung Nr. P 3818040.5 vom 27.05.1988).

Man erhält die Aminoguanidinoazine der Formel (IV), wenn man Sulfonylverbindungen der allgemeinen Formel (II) -oben - analog zum erfindungsgemäßen Verfahren (a) mit Hydrazin oder einem Hydrazin-Wasser-Addukt ("Hydrazin-Hydrat") gegebenenfalls in Gegenwart eines Verdünnungsmittels, wie z.B. Methylenchlorid, Methanol oder Ethanol, und gegebenenfalls in Gegenwart eines Trockenmittels, wie z.B. Natriumsulfat, bei Temperaturen zwischen -20° C und +80° C, vorzugsweise zwischen 0° C und 50° C, umsetzt.

Die beim erfindungsgemäßen Verfahren (b) weiter als Ausgangsstoffe zu verwendenden Sulfonsäurehalogenide sind durch die Formel (V) allgemein definiert.

In Formel (V) hat $R^2$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^2$ angegeben wurde und $X^1$ steht vorzugsweise für Fluor, Chlor oder

Brom, insbesondere für Chlor.

Als Beispiele für die Ausgangsstoffe der Formel (V) seien genannt:
2-Methoxycarbonyl- und 2-Ethoxycarbonyl- sowie 4-Methoxycarbonyl- und 4-Ethoxycarbonyl-benzolsulfonsäurechlorid.

Die Ausgangsstoffe der Formel (V) sind - wie auch die Ausgangsverbindung der Formel (VI) bekannt und/oder können nach an sich bekannten Verfahren hergestellt werden (vgl. EP-A 173320 und EP-A 173321).

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methylisopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (b) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Ka lium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen -100° C und +100° C, vorzugsweise bei Temperaturen zwischen -70° C und +70° C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel, gegebenenfalls in Gegenwart eines Säureakzeptors, durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Die beim erfindungsgemäßen Verfahren (c) als Ausgangsstoffe zu verwendenden substituierten Arylsulfonylaminoguanidinoazine sind durch die Formel (I) mit der Maßgabe, daß darin $R^2$ für gegebenenfalls substituiertes Alkoxy steht, allgemein definiert. In diesem Fall haben $R^1$, $R^3$, X, Y und Z vorzugsweise bzw. insbesondere diejenigen Bedeutungen, die bereits oben im Rahmen der Beschreibung der erfindungsgemäßen Verbindungen der Formel (I) als bevorzugt bzw. als besonders bevorzugt für $R^1$, $R^3$, X, Y und Z angegeben wurden und $R^2$ steht vorzugsweise für gegebenenfalls durch Fluor, Chlor, Methoxy oder Ethoxy substituiertes $C_1$-$C_4$-Alkoxy, insbesondere für Methoxy, Ethoxy, Propoxy, Isopropoxy, 2-Chlor-ethoxy, 2-Methoxy-ethoxy oder 2-Ethoxy-ethoxy.

Die beim erfindungsgemäßen Verfahren (c) als Ausgangsstoffe einzusetzenden Verbindungen der Formel (I) sind erfindungsgemäße, neue Verbindungen; sie können nach den erfindungsgemäßen Verfahren (a) oder (b) hergestellt werden.

Wäßrige Alkalilaugen, die beim erfindungsgemäßen Verfahren (c) eingesetzt werden können, sind Lösungen von Alkalimetallhydroxiden, wie z.B. von Lithiumhydroxid, Natriumhydroxid oder Kaliumhydroxid, in Wasser. Vorzugsweise wird wäßrige Natronlauge beim erfindungsgemäßen Verfahren (c) eingesetzt.

Ammoniak kann beim erfindungsgemäßen Verfahren (c) als Gas oder in Lösung, vorzugsweise in wäßriger Lösung eingesetzt werden.

Gegebenenfalls substituierte Alkylamine, welche beim erfindungsgemäßen Verfahren (c) eingesetzt werden können, sind vorzugsweise Alkylamine mit 1 bis 4 Kohlenstoffatomen, welche gegebenenfalls durch Fluor, Chlor, Methoxy oder Ethoxy substituiert sind, insbesondere Methylamin, Ethylamin, Propylamin und Isopropylamin.

Das erfindungsgemäße Verfahren (c) wird gegebenenfalls unter Verwendung von Verdünnungsmitteln durchgeführt. Als Verdünnungsmittel kommen dabei neben Wasser und Alkoholen, wie z.B. Methanol,

Ethanol und Isopropanol praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methylisobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Die Reaktionstemperaturen können bei dem erfindungsgemäßen Verfahren (c) in einem größeren Bereich variiert werden. Im allgemeinen arbeitet man bei Temperaturen zwischen 0 °C und 120 °C, vorzugsweise bei Temperaturen zwischen 20 °C und 100 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) setzt man auf 1 Mol Ausgangsverbindung der Formel (I) im allgemeinen zwischen 1 und 10 Mol, vorzugsweise zwischen 1 und 5 Mol,Alkalihydroxid, Ammoniak oder Amin ein.

Im allgemeinen wird die Ausgangsverbindung der Formel (I) bei Raumtemperatur, gegebenenfalls in einem Verdünnungsmittel, vorgelegt und die wäßrige Alkalilauge, das Ammoniak oder das Amin wird, gegebenenfalls bei erhöhter Temperatur, zudosiert. Das Reaktionsgemisch wird bis zum Ende der Umsetzung gerührt und nach üblichen Methoden aufgearbeitet (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage:

Aromaten, wie Xylol, Toluol, oder Alkylnaphthaline, chlorierte Aromaten und chlo rierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitrobenzoesäure (ACIFLUORFEN); Chloressigsäure-N-(methoxymethyl)-2,6-diethylanilid (ALACHLOR); Methyl-6,6-dimethyl-2,4-dioxo-3-[1-(2-propenyloxyamino)-butyliden]-cyclohexancarboxylat (ALLOXYDIM); 2-Chlor-4-ethylamino-6-isopropylamino-1,3,5-triazin (ATRAZIN); 2-[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl-oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl-oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N′-(3-trifluormethylphenyl)- harnstoff (FLUOMETURON); [(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N′-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 1-(3-Trifluormethylphenyl)-4-methylamino-5-chlor-6-pyridazon (NORFLURAZON); 4-(Di-n-propylamino)-3,5-dinitrobenzolsulfonamid (ORYZALIN); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); O-(6-Chlor-3-phenyl-pyridazin-4-yl)-S-octyl-thiocarbonat (PYRIDA TE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); S-[(4-Chlorphenyl)-methyl]-N,N-diethyl-thiocarbamat (THIOBENCARB); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele:

### Beispiel 1

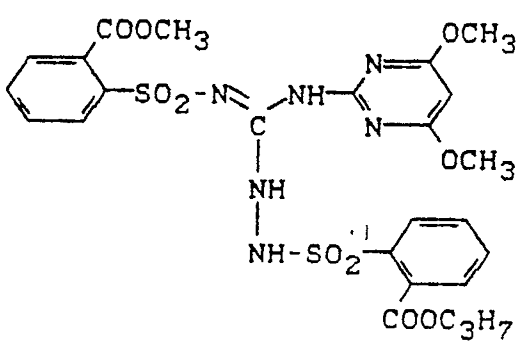

(Verfahren (b))

Eine Mischung aus 8,2 g (0,02 Mol) N′-(4,6-Dimethoxypyrimidin-2-yl)-N″-amino-N‴-(2-methoxycarbonyl-phenylsulfonyl)-guanidin, 2,5 g (0,022 Mol) Diazabicyclo-[2,2,2]-octan (DABCO) und 100 ml Methylenchlorid

wird auf -70° C abgekühlt und dazu wird unter Rühren eine Lösung von 5,2 g (0,02 Mol) 2-Propoxycarbonyl-benzolsulfonsäurechlorid in 20 ml Methylenchlorid tropfenweise gegeben. Nach Entfernen des Kühlbades wird das Reaktionsgemisch 12 Stunden gerührt, mit 100 ml Methylenchlorid verdünnt und mit 50 ml 1N-Salzsäure geschüttelt. Das hierbei kristallin angefallene Produkt wird durch Absaugen isoliert.

Man erhält 7,9 g (62% der Theorie) N′-(4,6-Dimethoxypyrimidin-2-yl)-N″-(2-propoxycarbonyl-phenylsulfonylamino)-N‴-(2-methoxycarbonyl-phenylsulfonyl)-guanidin vom Schmelzpunkt 180° C.

Beispiel 2

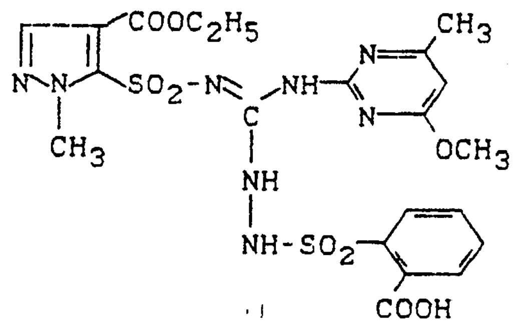

(Verfahren (b))

4,6 g (0,025 Mol) Sulfobenzoesäureanhydrid werden unter Rühren zu einer Mischung aus 10,3 g (0,025 Mol) N′-(4-Methoxy-6-methyl-pyrimidin-2-yl)-N″-amino-N‴-(1-methyl-4-ethoxycarbonyl-pyrazol-5-yl-sulfonyl)-guanidin und 100 ml Acetonitril gegeben und das Reaktionsgemisch wird noch 3 Stunden bei 20° C gerührt. Das hierbei kristallin angefallene Produkt wird dann durch Absaugen isoliert.

Man erhält 5,0 g (34% der Theorie) N′-(4-Methoxy-6-methyl-pyrimidin-2-yl)-N″-(2-carboxy-phenylsulfonylamino)-N‴-(1-methyl-4-ethoxycarbonylpyrazol-5-yl-sulfonyl)-guanidin vom Schmelzpunkt 228° C Zers..

Analog zu den Beispielen 1 und 2 und entsprechend der allgemeinen Beschreibung der erfindungsgemäßen Herstellungsverfahren können beispielsweise auch die in der nachstehenden Tabelle 3 aufgeführten Verbindungen der Formel (I) hergestellt werden.

**Tabelle 3:** Beispiele für die Verbindungen der Formel (I)

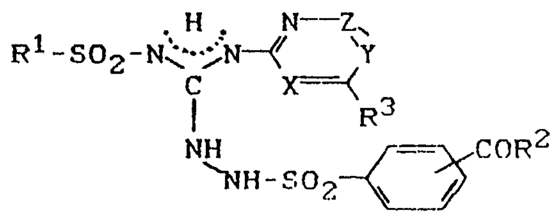

(I)

| Bsp.-Nr. | $R^1$ | Position $-COR^2$ | $R^3$ | X | Y | Z | Schmelzpunkt = Schmp. (°C) |
|---|---|---|---|---|---|---|---|
| 3 | 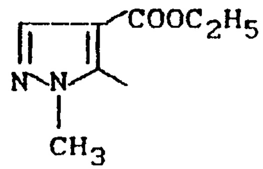 | (2-)$COOCH_3$ | $CH_3$ | N | CH | $C-CH_3$ | 157 |
| 4 | 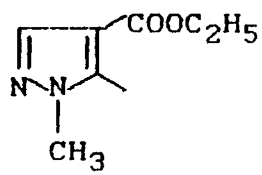 | (2-)$COOCH_3$ | $CH_3$ | N | CH | $C-OCH_3$ | 143 |
| 5 | 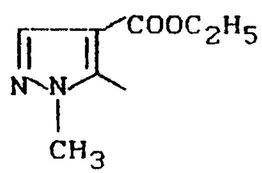 | (2-)$COOCH_3$ | $OCH_3$ | N | CH | $C-OCH_3$ | 177 |

Tabelle 3 - Fortsetzung

| Bsp.-Nr. | $R^1$ | Position $-COR^2$ | $R^3$ | X | Y | Z | Schmp. (°C) |
|---|---|---|---|---|---|---|---|
| 6 | 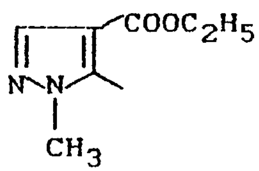 | (2-)$COOC_2H_5$ | $CH_3$ | N | CH | $C-OCH_3$ | 167 |
| 7 | 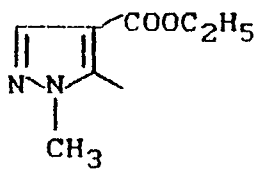 | (2-)$COOC_2H_5$ | $OCH_3$ | N | CH | $C-OCH_3$ | 167 |
| 8 | 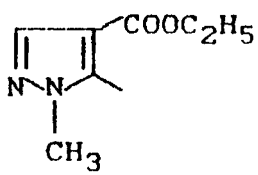 | (2-)$COOC_2H_5$ | $CH_3$ | N | CH | $C-CH_3$ | 163 |
| 9 | 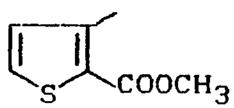 | (2-)$COOCH_3$ | $CH_3$ | N | CH | $C-CH_3$ | 199 |
| 10 | 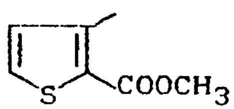 | (2-)$COOCH_3$ | $OCH_3$ | N | CH | $C-OCH_3$ | 193 |

Tabelle 3 - Fortsetzung

| Bsp.-Nr. | $R^1$ | Position $-COR^2$ | $R^3$ | X | Y | Z | Schmp. (°C) |
|---|---|---|---|---|---|---|---|
| 11 | 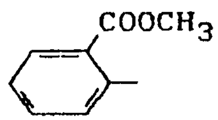 | (2-)$COOC_3H_7$-iso | $CH_3$ | N | CH | $C-CH_3$ | 159 |
| 12 | 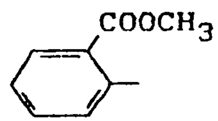 | (2-)$COOC_2H_5$ | $CH_3$ | N | CH | $C-CH_3$ | 199 |
| 13 | 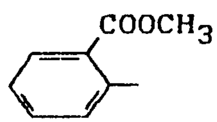 | (2-)$COOC_3H_7$-n | $CH_3$ | N | CH | $C-CH_3$ | 161 |
| 14 | 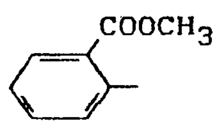 | (2-)$COOCH_2CH_2Cl$ | $CH_3$ | N | CH | $C-CH_3$ | 158 |
| 15 | 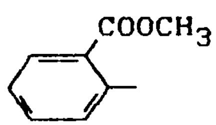 | (2-)$COOCH_2CH_2OC_2H_5$ | $CH_3$ | N | CH | $C-CH_3$ | 100 |
| 16 | 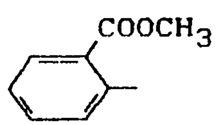 | (2-)$COOCH_2CH_2OC_2H_5$ | $OCH_3$ | N | CH | $C-OCH_3$ | 194 |

Tabelle 3 - Fortsetzung

| Bsp.-Nr. | $R^1$ | Position $-COR^2$ | $R^3$ | X | Y | Z | Schmp. (°C) |
|---|---|---|---|---|---|---|---|
| 17 | 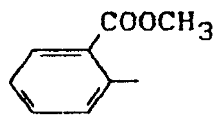 | (2-)$COOC_3H_7$-iso | $OCH_3$ | N | CH | $C-OCH_3$ | 192 |
| 18 | 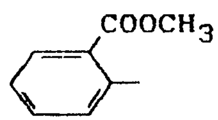 | (2-)$COOCH_2CH_2Cl$ | $OCH_3$ | N | CH | $C-OCH_3$ | 206 |
| 19 | 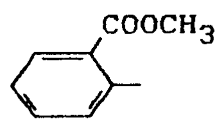 | (2-)COOH | $CH_3$ | N | CH | $C-CH_3$ | 208 |
| 20 | 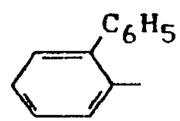 | (2-)$COOCH_3$ | $OCH_3$ | N | N | $C-OCH_3$ | 172 |
| 21 | 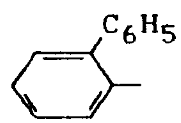 | (2-)$COOCH_3$ | $CH_3$ | N | CH | $C-CH_3$ | 142 |
| 22 | 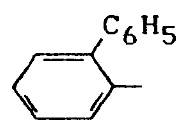 | (2-)$COOCH_3$ | $OCH_3$ | N | CH | $C-OCH_3$ | 187 |

<u>Tabelle 3</u> - Fortsetzung

| Bsp.-Nr. | $R^1$ | Position $-COR^2$ | $R^3$ | X | Y | Z | Schmp. (°C) |
|---|---|---|---|---|---|---|---|
| 23 | 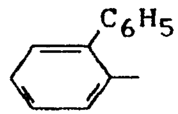 | (2-)$COOCH_3$ | $CH_3$ | N | N | C-$OCH_3$ | 139 |
| 24 | 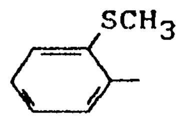 | (2-)$COOCH_3$ | $CH_3$ | N | CH | C-$CH_3$ | 177 |
| 25 | 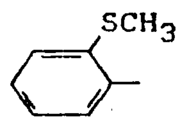 | (2-)$COOCH_3$ | $OCH_3$ | N | CH | C-$OCH_3$ | 143 |
| 26 | 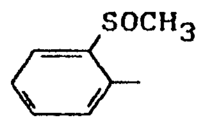 | (2-)$COOCH_3$ | $OCH_3$ | N | CH | C-$OCH_3$ | 194 |
| 27 | 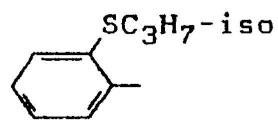 | (2-)$COOCH_3$ | $OCH_3$ | N | CH | C-$OCH_3$ | 142 |
| 28 | 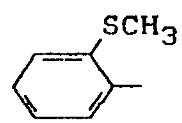 | (2-)$COOCH_3$ | $OCH_3$ | N | N | C-$OCH_3$ | 173 |

Tabelle 3 - Fortsetzung

| Bsp.-Nr. | $R^1$ | Position $-COR^2$ | $R^3$ | X | Y | Z | Schmp. (°C) |
|---|---|---|---|---|---|---|---|
| 29 | 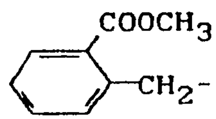 | (2-)$COOCH_3$ | $OCH_3$ | N | CH | $C-OCH_3$ | 175 |
| 30 | 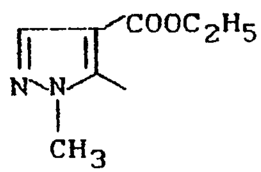 | (2)-$COOC_3H_7$-i | $CH_3$ | N | CH | $C-CH_3$ | 141 |
| 31 | 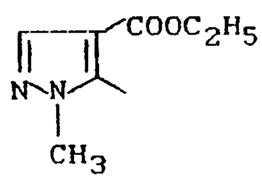 | (2)-$COOC_3H_7$-i | $OCH_3$ | N | CH | $C-CH_3$ | 159 |
| 32 | 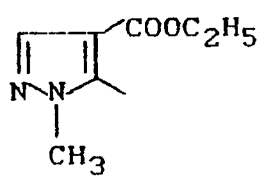 | (2)-$COOC_3H_7$-i | $OCH_3$ | N | CH | $C-OCH_3$ | 172 |
| 33 | 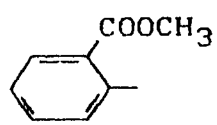 | (2)-$CONHCH(CH_3)_2$ | $CH_3$ | N | CH | $C-CH_3$ | 220 |

Tabelle 3 - Fortsetzung

| Bsp.-Nr. | $R^1$ | Position $-COR^2$ | $R^3$ | X | Y | Z | Schmp. (°C) |
|---|---|---|---|---|---|---|---|
| 34 | 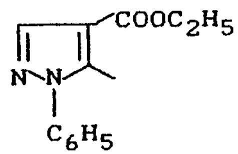 | (2)-$COOCH_3$ | $OCH_3$ | N | CH | C-$CH_3$ | 186 |
| 35 | 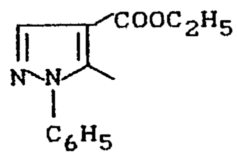 | (2)-$COOC_2H_5$ | $CH_3$ | N | CH | C-$CH_3$ | 149 |
| 36 | 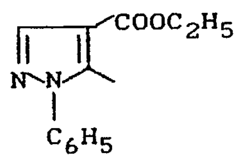 | (2)-$COOC_2H_5$ | $OCH_3$ | N | CH | C-$OCH_3$ | 176 |
| 37 | 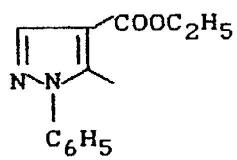 | (2)-$COOCH(CH_3)_2$ | $OCH_3$ | N | CH | C-$OCH_3$ | 165 |
| 38 | 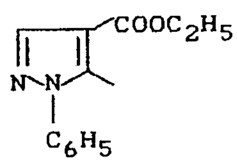 | (2)-$COOCH_3$ | $OCH_3$ | N | CH | C-$CH_3$ | 166 |

Tabelle 3 - Fortsetzung

| Bsp.-Nr. | $R^1$ | Position $-COR^2$ | $R^3$ | X | Y | Z | Schmp. (°C) |
|---|---|---|---|---|---|---|---|
| 39 | 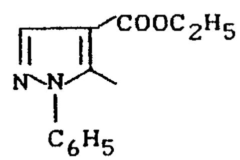 | (2)-$COOCH_3$ | $OCH_3$ | N | CH | $C-CH_3$ | 178 |
| 40 | 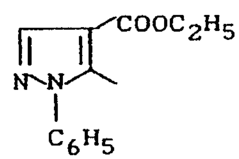 | (2)-$COOCH(CH_3)_2$ | $OCH_3$ | N | CH | $C-CH_3$ | 152 |
| 41 | 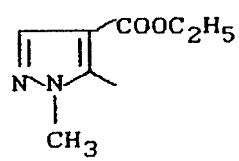 | (2)-$COOCH_3$ | $CH_3$ | N | CH | CH | 161 |
| 42 | 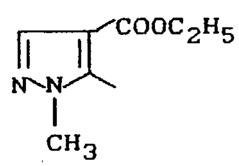 | (2)-$COOC_4H_9$-n | $OCH_3$ | N | CH | $C-CH_3$ | 141 |
| 43 | 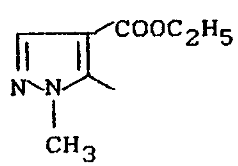 | (2)-$COOC_4H_9$-s | $OCH_3$ | N | CH | $C-CH_3$ | 158 |

Tabelle 3 - Fortsetzung

| Bsp.-Nr. | $R^1$ | Position $-COR^2$ | $R^3$ | X | Y | Z | Schmp. (°C) |
|---|---|---|---|---|---|---|---|
| 44 | 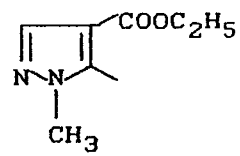 | (2)-$COOC_4H_9$-s | $OCH_3$ | N | CH | C-$OCH_3$ | 122 |
| 45 | 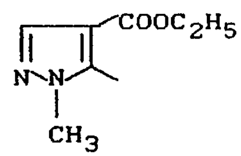 | (2)-$COOC_4H_9$-n | $CH_3$ | N | CH | C-$CH_3$ | 132 |
| 46 | 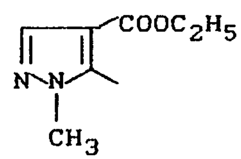 | (2)-$COOC_4H_9$-n | $OCH_3$ | N | CH | C-$OCH_3$ | 153 |
| 47 | 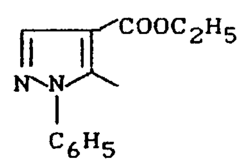 | (2)-$COOC_4H_9$-s | $OCH_3$ | N | CH | C-$CH_3$ | 154 |
| 48 | 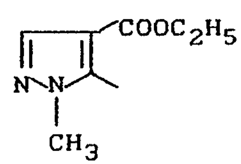 | (2)-$COOC_4H_9$-s | $CH_3$ | N | CH | C-$CH_3$ | 121 |

<u>Tabelle 3</u> - Fortsetzung

| Bsp.-Nr. | $R^1$ | Position $-COR^2$ | $R^3$ | X | Y | Z | Schmp. (°C) |
|---|---|---|---|---|---|---|---|
| 49 | 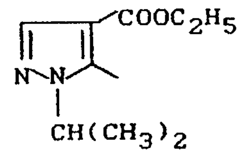 | (2)-$COOCH_3$ | $OCH_3$ | N | CH | C-$OCH_3$ | 170 |
| 50 | 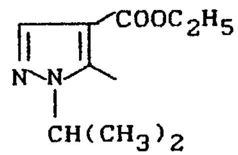 | (2)-$COOC_2H_5$ | $OCH_3$ | N | CH | C-$OCH_3$ | 188 |
| 51 | 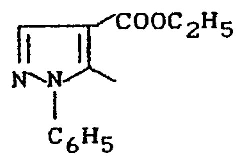 | (2)-$COOC_4H_9$-n | $CH_3$ | N | CH | C-$CH_3$ | 142 |
| 52 | 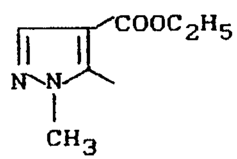 | (2)-$COOC_3H_7$-n | $OCH_3$ | N | CH | C-$CH_3$ | 148 |
| 53 | 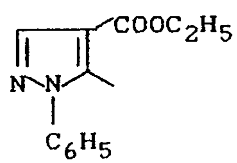 | (2)-$COOC_4H_9$-n | $OCH_3$ | N | CH | C-$OCH_3$ | 180 |

<u>Tabelle 3</u> - Fortsetzung

| Bsp.-Nr. | $R^1$ | Position $-COR^2$ | $R^3$ | X | Y | Z | Schmp. (° C) |
|---|---|---|---|---|---|---|---|
| 54 | 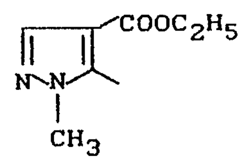 | (2)-$COOC_3H_7$-n | $CH_3$ | N | CH | $C-CH_3$ | 126 |
| 55 | 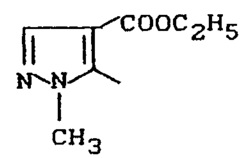 | (2)-$COOC_3H_7$-n | $OCH_3$ | N | CH | $C-OCH_3$ | 162 |
| 56 | 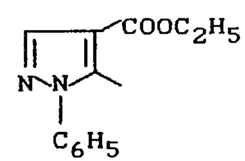 | (2)-$COOC_4H_9$-n | $OCH_3$ | N | CH | $C-CH_3$ | 107 |
| 57 | 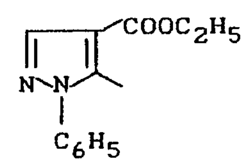 | (2)-$COOC_3H_7$-n | $OCH_3$ | N | CH | $C-CH_3$ | 182 |
| 58 | 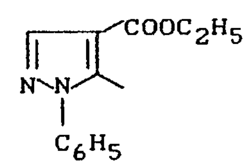 | (2)-$COOCH(CH_3)_2$ | $OCH_3$ | N | CH | $C-CH_3$ | 142 |

Tabelle 3 - Fortsetzung

| Bsp.-Nr. | $R^1$ | Position $-COR^2$ | $R^3$ | X | Y | Z | Schmp. (°C) |
|---|---|---|---|---|---|---|---|
| 59 | 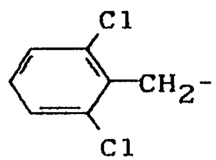 | (2)-$COOCH_3$ | $OCH_3$ | N | CH | $C-CH_3$ | 191 |
| 60 | 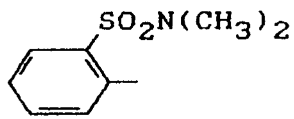 | (2)-$COOCH_3$ | $OCH_3$ | N | CH | $C-CH_3$ | 192 |
| 61 | 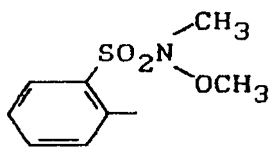 | (2)-$COOCH_3$ | $OCH_3$ | N | CH | $C-CH_3$ | 142 |
| 62 | 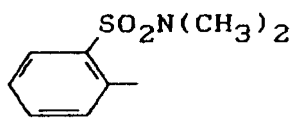 | (2)-$COOCH_3$ | $CH_3$ | N | CH | CH | 178 |
| 63 | 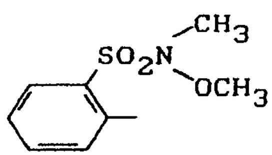 | (2)-$COOCH_3$ | $CH_3$ | N | CH | CH | 184 |

<u>Tabelle 3</u> - Fortsetzung

| Bsp.-Nr. | $R^1$ | Position $-COR^2$ | $R^3$ | X | Y | Z | Schmp. (°C) |
|---|---|---|---|---|---|---|---|
| 64 | 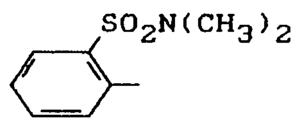 | (2)-$COOC_2H_5$ | $CH_3$ | N | CH | CH | 168 |
| 65 | 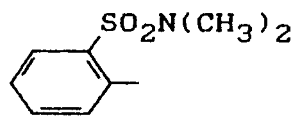 | (2)-$COOC_3H_7$-n | $CH_3$ | N | CH | CH | 163 |
| 66 | 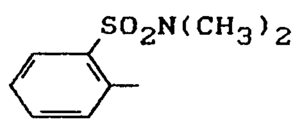 | (2)-$COOCH(CH_3)_2$ | $CH_3$ | N | CH | CH | 166 |
| 67 | 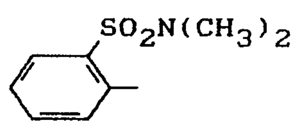 | (2)-$COOC_4H_9$-n | $CH_3$ | N | CH | CH | 165 |
| 68 | 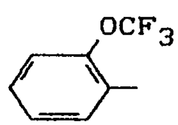 | (2)-$COOCH_3$ | $OCH_3$ | N | CH | C-$CH_3$ | 184 |
| 69 | 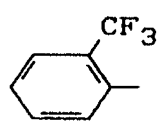 | (2)-$COOCH_3$ | $OCH_3$ | N | CH | C-$OCH_3$ | 189 |

Tabelle 3 - Fortsetzung

| Bsp.-Nr. | $R^1$ | Position $-COR^2$ | $R^3$ | X | Y | Z | Schmp. (°C) |
|---|---|---|---|---|---|---|---|
| 70 | 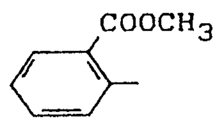 | (2)-$CONHCH_2CH_2OCH_3$ | $CH_3$ | N | CH | $C-CH_3$ | 184 |
| 71 | 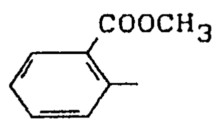 | (2)-$CONH(CH_2)_3OCH_3$ | $CH_3$ | N | CH | $C-CH_3$ | 166 |
| 72 | 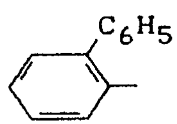 | (2)-$COOCH_3$ | $OCH_3$ | N | CH | $C-CH_3$ | 107 |
| 73 | 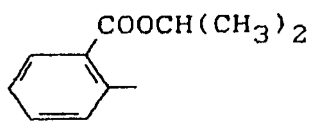 | (2)-$COOCH_3$ | $OCH_3$ | N | N | $C-OCH_3$ | 115 |
| 74 | 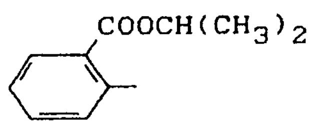 | (2)-$COOCH_3$ | $OCH_3$ | N | CH | $C-OCH_3$ | 164 |
| 75 | 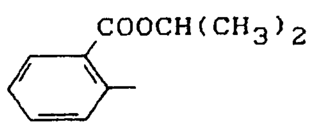 | (2)-$COOCH_3$ | $CH_3$ | N | CH | $C-CH_3$ | 159 |

Die Herstellung der in Tabelle 3 als Beispiel Nr. 19 aufgeführten Verbindung ist im Folgenden ausführlich beschrieben:

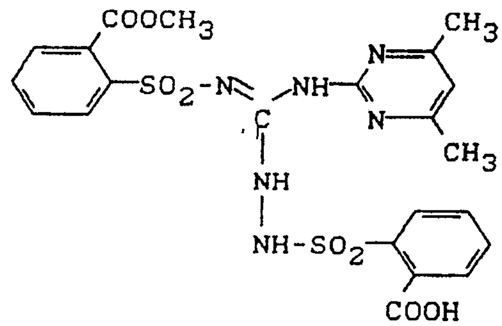

(Verfahren (c))

3,0 g (5,0 mMol) N'-(4,6-Dimethyl-pyrimidin-2-yl)-N''-(2-(2-Chlor-ethoxycarbonyl)-phenylsulfonylamino)-N'''-(2-methoxycarbonyl-phenylsulfonyl)-guanidin werden in 20 ml Wasser suspendiert und zu dieser Suspension wird bei 60° C tropfenweise 50%ige Natronlauge gegeben, wobei ein Überschreiten von pH 12 zu vermeiden ist. Die entstandene klare Lösung wird nach Abkühlen mit dreimal je 50 ml Chloroform ausgeschüttelt; die wäßrige Phase wird dann filtriert und durch Zugabe von konzentrierter Schwefelsäure pH 5 eingestellt. Der ausgefallene Feststoff wird abfiltriert, mit Wasser gewaschen, in Methylenchlorid aufgenommen, mit Natriumsulfat getrocknet und filtriert. Vom Filtrat wird das Lösungsmittel im Wasserstrahlvakuum sorgfältig abdestilliert.

Man erhält 1,0 g (33% der Theorie) N'-(4,6-Dimethylpyrimidin-2-yl)-N''-(2-Carboxy-phenylsulfonylamino)-N'''-(2-methoxycarbonyl-phenylsulfonyl)-guanidin vom Schmelzpunkt 208° C.

Die Herstellung der in Tabelle 3 als Beispiel Nr. 33 aufgeführten Verbindung ist im Folgenden ausführlich beschrieben:

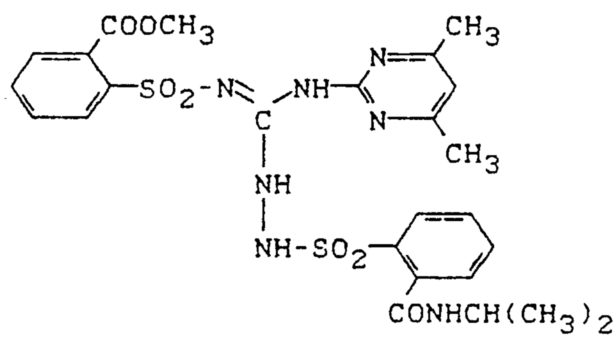

(Verfahren (c))

Eine Mischung aus 3,0 g (5,0 mMol) N'-(4,6-Dimethyl-pyrimidin-2-yl)-N''-(2-(2-chlor-ethoxycarbonyl)-phenylsulfonylamino)-N'''-(2-methoxycarbonyl-phenylsulfonyl)-guanidin, 1,5 g (25 mMol) Isopropylamin und 25 ml Tetrahydrofuran wird 24 Stunden bei 60° C gerührt und dann eingeengt. Der Rückstand wird durch Verreiben mit Methanol zur Kristallisation gebracht und das Produkt wird durch Absaugen isoliert.

Man erhält 1,5 g (50% der Theorie) N'-(4,6-Dimethyl-pyrimidin-2-yl)-N''-(2-Isopropylaminocarbonyl-phenylsulfonylamino)-N'''-(2-methoxycarbonyl-phenylsulfonyl)guanidin vom Schmelzpunkt 220° C.

Ausgangsstoffe der Formel (IV)

Beispiel (IV-1)

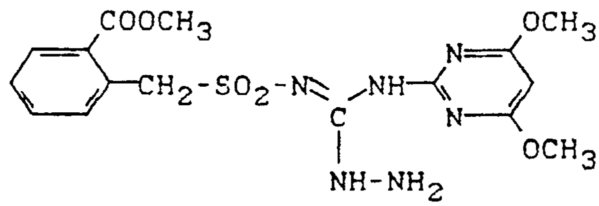

1,3 g (0,025 Mol) Hydrazinhydrat werden bei anfangs 20° C zu einer Suspension von 15,9 g (0,025 Mol) N'-(4,6-Dimethoxy-pyrimidin-2-yl)-N''-methoxy-N''-(2-methoxycarbonyl-phenylsulfonyl)-N'''-(2-methoxycarbonyl-benzylsulfonyl)-guanidin in 100 ml Methanol unter Rühren gegeben, wobei sich die Reaktionsmischung auf 30° C erwärmt und eine klare Lösung entsteht. Das nach vierstündigem Rühren bei 20° C bis 30° C kristallin abgeschiedene Produkt wird durch Absaugen isoliert.

Man erhält 9,5 g (89% der Theorie) N'-(4,6-Dimethoxypyrimidin-2-yl)-N''-amino-N'''-(2-methoxycarbonyl-benzylsulfonyl)-guanidin vom Schmelzpunkt 166° C.

Anwendungsbeispiele:

In den folgenden Anwendungsbeispielen werden die nachstehend aufgeführten Verbindungen als Vergleichssubstanzen herangezogen:

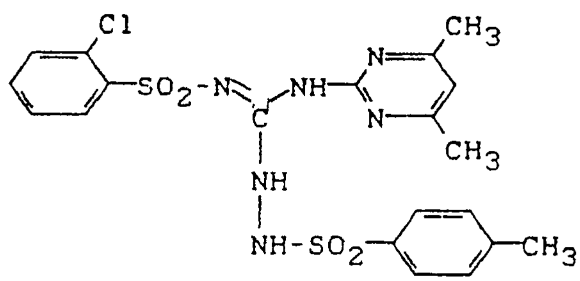

(A)

N'-(4,6-Dimethyl-pyrimidin-2-yl)-N''-(4-methyl-phenylsulfonylamino)-N'''-(2-chlor-phenylsulfonyl)-guanidin (bekannt aus EP-A 121082).

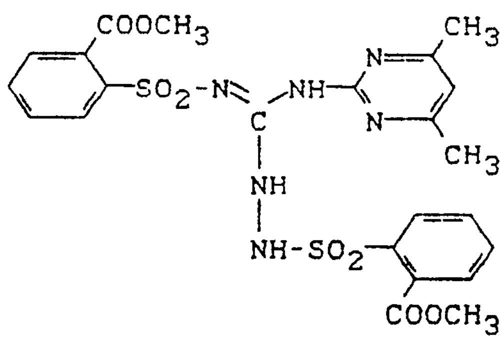

(B)

N'-(4,6-Dimethyl-pyrimidin-2-yl)-N''-(2-methoxycarbonylphenylsulfonylamino)-N'''-(2-methoxycarbonyl-phenylsulfonyl)-guanidin
(bekannt aus EP-A 302378).

Beispiel A

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche bessere Nutzpflanzenselektivität gegenüber dem Stand der Technik zeigen in diesem Test z.B. die Verbindungen gemäß den Herstellungsbeispielen (1), (4), (5), (7), (10), (17), (18), (19), (25), (26), (29), (67).

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutlich bessere Nutzpflanzenselektivität gegenüber dem Stand der Technik zeigen in diesem Test z.B. die Verbindungen gemäß den Herstellungsbeispielen (4), (5), (7), (10), (18), (19), (25), (26), (67).

## Ansprüche

1. Substituierte Arylsulfonylaminoguanidinoazine der allgemeinen Formel (I),

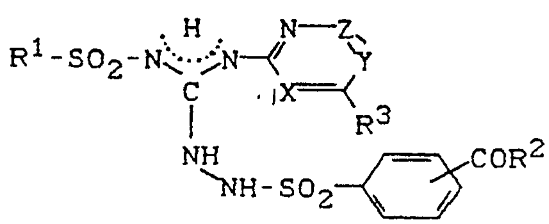

(I)

in welcher
$R^1$ für jeweils gegebenenfalls substituiertes Aryl, Aralkyl oder Heteroaryl steht,
$R^2$ für Hydroxy, Amino oder für jeweils gegebenenfalls substituiertes Alkoxy oder Alkylamino steht,
$R^3$ für Wasserstoff, Halogen, Hydroxy, Alkyl, Halogenalkyl, Alkoxyalkyl, Alkoxy, Halogenalkoxy, Alkylthio, Halogenalkylthio, Amino, Alkylamino oder Dialkylamino steht,
X für Stickstoff oder eine -CH-Gruppierung steht,
Y für Stickstoff oder eine -$CR^4$-Gruppierung steht, worin
$R^4$ für Wasserstoff, Halogen, Cyano, Alkyl, Formyl, Alkyl-carbonyl oder Alkoxy-carbonyl steht, und

Z für Stickstoff oder eine -$CR^5$-Gruppierung steht, worin
$R^5$ für Wasserstoff, Halogen, Hydroxy, Alkyl, Alkoxy, Alkylthio, Alkylamino oder Dialkylamino steht, ausgenommen die Verbindung N'-(4,6-Dimethyl-pyrimidin-2-yl)-N''-(2-methoxycarbonyl-phenylsulfonylamino)-N'''-(2-methoxycarbonyl-phenylsulfonyl)-guanidin.

2. Verfahren zur Herstellung von substituierten Arylsulfonylaminoguanidinoazinen der Formel (I) gemäß Anspruch 1, dadurch gekennzeichnet, daß man

(a) Sulfonylverbindungen der allgemeinen Formel (II)

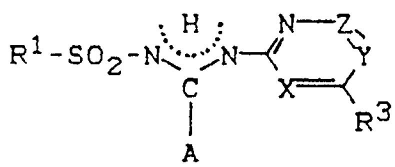

(II)

in welcher
$R^1$, $R^3$, X, Y und Z die in Anspruch 1 angegebenen Bedeutungen haben und
A für eine der nachstehend angegebenen Abgangsgruppen

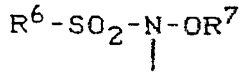 oder $-Q-R^8$ steht,

worin
$R^6$ die oben für $R^1$ angegebene Bedeutung hat, aber nicht in jedem Einzelfall mit $R^1$ identisch sein muß,
$R^7$ für Alkyl, Alkenyl oder Aralkyl steht,
$R^8$ für Alkyl, Aralkyl oder Aryl steht und
Q für Sauerstoff oder Schwefel steht,
mit Sulfonsäurehydraziden der allgemeinen Formel (III)

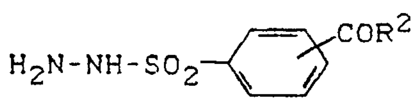

(III)

in welcher
$R^2$ die in Anspruch 1 angegebene Bedeutung hat,
gegebenenfalls in Gegenwart eines Verdünnungsmittels umsetzt, oder daß man

(b) Aminoguanidinoazine der allgemeinen Formel (IV)

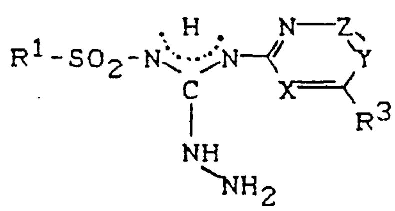

(IV)

in welcher
$R^1$, $R^3$, X, Y und Z die in Anspruch 1 angegebenen Bedeutungen haben,
mit Sulfonsäurehalogeniden der allgemeinen Formel (V)

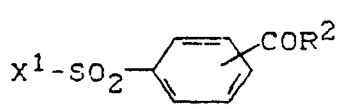

(V)

in welcher
$R^2$ die in Anspruch 1 angegebene Bedeutung hat und

$X^1$ für Halogen steht,
oder mit Sulfobenzoesäureanhydrid der Formel (VI)

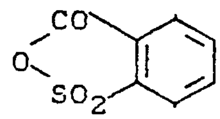

(VI)

gegebenenfalls in Gegenwart eines Verdünnungsmittels und gegebenenfalls in Gegenwart eines Säureakzeptors umsetzt, oder daß man

(c) substituierte Arylsulfonylaminoguanidinoazine der allgemeinen Formel (I) gemäß Anspruch 1, in welcher $R^2$ für gegebenenfalls substituiertes Alkoxy steht und $R^1$, $R^3$, X, Y und Z die oben angegebenen Bedeutungen haben,
mit wäßrigen Alkalilaugen oder mit Ammoniak oder mit gegebenenfalls substituierten Alkylaminen gegebenenfalls in Gegenwart eines Verdünnungsmittels umsetzt.

3. Herbizide Mittel, gekennzeichnet durch einen Gehalt an mindestens einem substituierten Arylsulfonylaminoguanidinoazin der Formel (I) gemäß Anspruch 1.

4. Verfahren zur Bekämpfung von Unkräutern, dadurch gekennzeichnet, daß man substituierte Arylsulfonylaminoguanidinoazine der Formel (I) gemäß Anspruch 1 auf Unkräuter und/oder ihren Lebensraum einwirken läßt.

5. Verwendung von substituierten Arylsulfonylaminoguanidinoazinen der Formel (I) gemäß Anspruch 1 zur Bekämpfung von Unkräutern.

6. Verfahren zur Herstellung von herbiziden Mitteln, dadurch gekennzeichnet, daß man substituierte Arylsulfonylaminoguanidinoazine der Formel (I) gemäß Anspruch 1 mit Streckmitteln und/oder oberflächenaktiven Mitteln vermischt.